# EUROPEAN PATENT APPLICATION

(11) **EP 4 596 540 A1**
(43) Date of publication of application: **06.08.2025**
(21) Application number: 23940933.7
(22) Date of filing: 05.09.2023
(51) Int. Cl.: C07D 405/04, C07D 407/02, C07H 19/01, A61K 31/712, A61P 43/00

(54) **CARBOCYCLIC NUCLEOSIDE, OLIGONUCLEOTIDE, PREPARATION METHOD THEREFOR, AND MEDICAL USES THEREOF**

(30) Priority: 21.07.2023 CN 202310902267; 04.09.2023 CN 202311130197
(71) Applicant: Redfield Pharmaceutical Inc., Nanjing, Jiangsu 210033 (CN); Redfield Biotech Limited, Nanjing, Jiangsu 210033 (CN)
(72) Inventor: CHEN, Lulu, Nanjing, Jiangsu 210033 (CN); CHENG, Guang, Nanjing, Jiangsu 210033 (CN); DONG, Ji, Nanjing, Jiangsu 210033 (CN); ZHANG, Tianlong, Nanjing, Jiangsu 210033 (CN); ZHOU, Tong, Nanjing, Jiangsu 210033 (CN); HU, Xionglin, Nanjing, Jiangsu 210033 (CN); SHENG, Xiaoning Christopher, Nanjing, Jiangsu 210033 (CN)
(74) Representative: Murgitroyd & Company
(86) International application number: PCT/CN2023/117016
(87) International publication number: WO 2025/015676

(57) **Abstract**

Disclosed are a carbocyclic nucleoside, an oligonucleotide and preparation methods therefor and medical uses thereof, and the carbocyclic nucleoside is a compound or salt shown in the following Formula (I). The oligonucleotide or pharmaceutically acceptable salt thereof provided in the present disclosure can be used for preparing a drug for gene therapy, gene vaccination, antisense therapy, interfering RNA, or nucleic acid transfer. The oligonucleotide in the present disclosure has high binding affinity to single-stranded RNA, nuclease resistance and plasma stability.

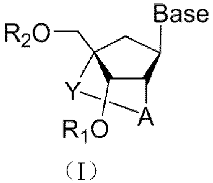

## Description

### TECHNICAL FIELD

The present disclosure relates to a nucleoside and a nucleotide and, as well as preparation methods therefor and medical uses thereof, and particularly relates to a carbocyclic nucleoside, an oligonucleotide and preparation methods therefor and medical uses thereof.

### BACKGROUND

Nucleic acids are one of the basic substances of life, and include deoxyribonucleic acid (DNA) and ribonucleic acid (RNA). Nucleotides are monomers that constitute nucleic acid molecules, and they are a compound composed of nitrogenous bases, pentoses and phosphates, and are phosphate esters of nucleosides. Nucleic acid therapy refers to the use of modified single-stranded or double-stranded nucleotides as drugs or vaccines applied to a human body, with a basic composition being a nucleotide sequence, which acts on mRNA through complementary base pairing and inhibits the expression of target protein by gene silencing, so as to achieve the effects of disease treatment and prevention. Nucleic acid-based therapeutic modalities include antisense oligonucleotide (ASO), small interfering RNA (siRNA), microRNA (miRNA), small activating RNA (saRNA), aptamer, messenger RNA (mRNA), ribozymes, antibody-oligonucleotide conjugate (AOC), plasmid DNA, CRISPR/Cas9, and the like. A short-chain nucleotide containing about 20 bases are called oligonucleotide, which mainly includes ASO, siRNA, aptamer, miRNA, saRNA, and the like. Specifically, ASO is a synthetic single-stranded oligonucleotide usually composed of 12-30 nucleotides, it binds to target mRNA or pre-mRNA upon Watson-Crick complementary base pairing principles, induces Ribonuclease H (RNase H) to degrade an RNA strand, or affects the processing and splicing of pre-mRNA through steric hindrance, thereby regulating gene expression. As a first ASO drug that was commercialized, Fomivirsen was approved by the FDA in 1998. At present, 10 ASO drugs have been approved for marketing worldwide. siRNA is a double-stranded oligonucleotide containing a sense strand and an antisense strand. It binds to the RNA-induced silencing complex (RISC) in the cytoplasm. Under the action of argonaute 2 protein, the double strand dissociates, specifically, the sense strand is cleaved and left, while the antisense strand in the RISC binds to mRNA, resulting in mRNA degradation and regulating gene expression. In 2018, the FDA approved a first siRNA drug, Patisiran. By 2023, other five siRNA drugs had been approved in succession for marketing. Aptamer is single-stranded nucleic acid, usually with 25-50 bases in length and having unique three-dimensional conformations. It can target biological components such as protein on a surface or inside cell of diseased tissue and block a protein function through steric hindrance. Aptamer can serve as an antibody analog. Pegaptanib is an aptamer drug approved by the FDA in 2006. There are several aptamer drugs in clinical research stages at present. miRNA is a non-coding RNA with a length of 19-24 nucleotides. After binding to mRNA in a cell, miRNA performs post-transcriptional regulation, inhibits protein translation and regulates the expression of target gene. saRNA is a short double-stranded oligonucleotide with a structure similar to that of siRNA, it is capable of activating gene transcription, increasing mRNA expression and upregulating target protein by recruiting an endogenous transcription complex to a target gene, thus exerting therapeutic effects.

As early as half a century ago, artificially synthesized oligonucleotides were used to regulate targeted gene. However, unmodified oligonucleotides have disadvantages such as poor stability, easily degradation by endogenous nucleases in the blood, quickly clearing by the liver and kidneys, short half-lives, and low bioavailability, making them difficult to be developed as drugs. Resistance of oligonucleotides to enzymatic degradation can be improved through chemical modification, and affinity to a target can be accordingly increased, a first oligonucleotide drug was launched in 1998. Chemical modifications of nucleic acids mainly include phosphate backbone modification, base modification, ribose modification, and the like. Specifically, a most typical backbone modification is thiophosphorylation, where one non-bridging oxygen atom in a phosphate group of a nucleotide chain is replaced by a sulfur atom, such that nuclease resistance degradation thereof is greatly increased. Another backbone modification involves the substitution of 3' oxygen atom in a ribose ring with 3' amine, and thiocarbamate and phosphoryl imide salt exhibit high affinity and nuclease resistance. Base modification usually involves the introduction of functional groups such as carboxyl or amino at a 5' position of a pyrimidine base and a 8' position of a purine base. Ribose modification mainly occurs at a 2' position, including 2'-O-methyl, 2'-O-methoxyethyl, 2'-F and other modifications, which can improve affinity to varying degrees and increase nuclease resistance. Linking 4' and 2' positions of ribose to form bicyclic systems such as locked nucleic acid (LNA) or bridged nucleic acid (BNA), which is conducive to improving affinity greatly. Drug researchers have been making great efforts to improve druggability through various modifications, contributing to continuous development and marketing of new nucleic acid drugs. However, no ideal molecule is available yet, and there is still room for improvement in the affinity, nuclease resistance, and in vivo stability of existing nucleic acid drugs.

### SUMMARY

Objectives of the present disclosure: one objective of the present disclosure is to provide a carbocyclic nucleoside and an oligonucleotide having high binding affinity to single-stranded nucleic acid (RNA or DNA), nuclease resistance and plasma stability; another objective of the present disclosure is to provide an oligonucleotide and a preparation method therefor; yet another objective of the present disclosure is to provide a pharmaceutical composition; and still another objective of the present disclosure is to provide uses of the oligonucleotide in the preparation of a drug for gene therapy, gene vaccination, antisense therapy, interfering RNA, or nucleic acid transfer.

Technical solution: the present disclosure provides a compound or salt represented by the following Formula (I): 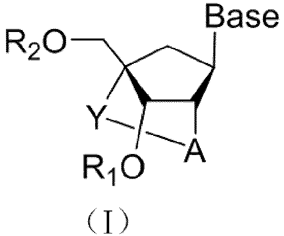
where R₁ and R₂ independently represent a hydrogen atom, an alkyl group containing 1-6 carbon atoms, a phenyl group, a (C₁₋₃) alkyl-phenyl group, a heterocycle, or a (C₁₋₃) alkyl-heterocycle, a hydroxyl protecting group, a silicon group containing one or more substituents, a phosphate group containing a protecting group for oligonucleotide synthesis, phosphoester, thiophosphate, chiral thiophosphate, phosphate triester, aminoalkyl phosphate triester, and alkyl phosphate ester;
A is O, S, or NR₃; R₃ is a hydrogen atom, an alkyl group containing 1-6 carbon atoms, a halogenated alkyl group containing 1-6 carbon atoms, -C(O)R₄, -C(O)OR₄, or -C(O)N(R₄)₂; and R₄ is H, or an alkyl group containing 1-6 carbon atoms or a halogenated alkyl group containing 1-6 carbon atoms;
Y is C=O, CHR₅, or CHOR₅; and R₅ is H, or an alkyl group containing 1-6 carbon atoms or a halogenated alkyl group containing 1-6 carbon atoms; and
Base is a base.

As a further improvement of the above technical solution, Formula (I) is represented by any one of the following Formulae (I-1) - (I-4):

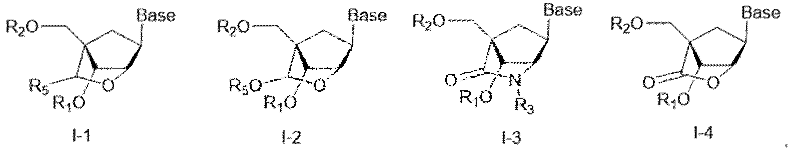

Preferably, Base is selected from any of the following groups:

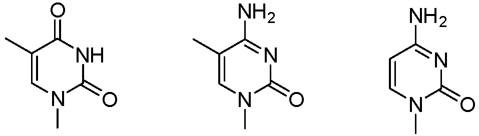

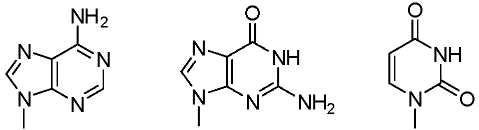

Preferably, the alkyl group containing 1-6 carbon atoms is a straight-chain alkyl group, a branched alkyl group, a cycloalkyl group or a (C₁₋₄) alkyl group-(C₃₋₇) cycloalkyl group.

Preferably, the compound represented by Formula (I) has any of the structures shown in the following formula:

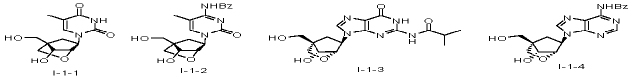

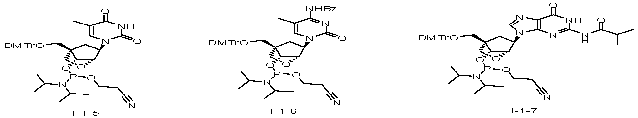

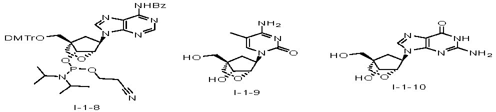

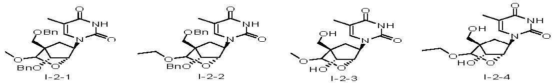

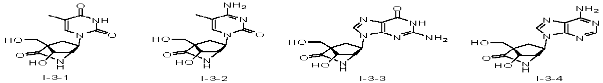

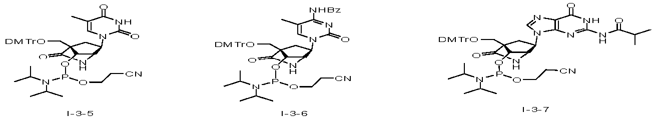

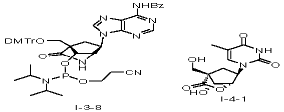

Further, the present disclosure provides an oligonucleotide or pharmaceutically acceptable salt thereof, which contains at least one of the structures shown in Formulae (II)-(IV): 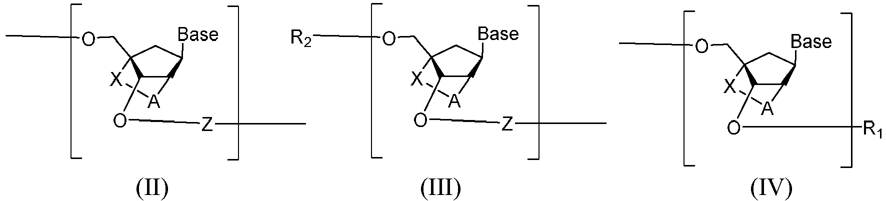 where Z is phosphoester, thiophosphate, chiral thiophosphate, phosphate triester, aminoalkyl phosphate triester, and alkyl phosphate ester.

In the above solution, the oligonucleotide is a linear polymer compound formed by covalent linkage of adjacent nucleotides through phosphate groups. alternatively, the oligonucleotide can be a spacer polymer formed by linkage of phosphoester, thiophosphate, chiral thiophosphate, phosphate triester, aminoalkyl phosphate triester, and alkyl phosphate ester with DNA or RNA.

As described in the present disclosure, the oligonucleotide preferably contains approximately 12-50 nucleotides and preferably a backbone containing phosphorus atoms, such as phosphoester, thiophosphate, chiral thiophosphate, phosphate triester, aminoalkyl phosphate triester, or alkyl phosphate ester.

In the above oligonucleotide structure, the conformationally locked carbon-ring nucleoside can form polymers by linking with DNA through phosphoester, thiophosphate, chiral thiophosphate, phosphate triester, aminoalkyl phosphate triester, and alkyl phosphate ester; alternatively, the conformationally locked carbon-ring nucleoside can form polymers by linking with RNA through phosphoester, thiophosphate, chiral thiophosphate, phosphate triester, aminoalkyl phosphate triester, and alkyl phosphate ester; and alternatively, the conformationally locked carbon-ring nucleoside can form spacer polymers by linking with DNA and RNA through phosphoester, thiophosphate, chiral thiophosphate, phosphate triester, aminoalkyl phosphate triester, and alkyl phosphate ester.

The above solution provides a conformationally locked carbon-ring nucleoside for nucleic acid modification. The oligonucleotide is a synthetic nucleotide polymer having single-stranded or double-stranded structures. Oligonucleotide therapies include: regulating gene expression with antisense oligonucleotide designed by Watson-Crick base pairing; small interfering RNA (siRNA) that cleaves target transcripts using an RNA-induced silencing complex (RISC); aptamers that inhibit protein function by interacting with target proteins via three-dimensional structures thereof, and the like. Advantages of oligonucleotide therapies include simple design of drug molecules based on known genomic information, high target specificity, and reduced off-target toxicity.

In order to improve the stability and pharmacokinetic properties of oligonucleotide, chemically modified nucleic acids (phosphorothioate backbone) and sugar-modified nucleic acids, such as 2'-O-methyl, 2'-O-methoxyethyl, 2'-fluoro, locked nucleic acids (LNA), and bridged nucleic acids (BNA), have been successfully introduced. Oligonucleotide therapeutics are usually small in size, easily filtered by the kidneys, have poor blood pharmacokinetic properties and low concentrations in target tissues. The oligonucleotide developed in the present disclosure is expected to exhibit high binding affinity to the target gene while having a smaller polar surface, which can improve the blood pharmacokinetic properties.

Oligonucleotides in the present disclosure include antisense oligonucleotide (ASO), small interfering RNA (siRNA), microRNA (miRNA), small activating RNA (saRNA), aptamer, and the like. The conformationally locked carbon-ring nucleoside can be present at one or more positions of the oligonucleotide.

Further, the present disclosure provides a preparation method of the oligonucleotide or pharmaceutically acceptable salt thereof, including steps of synthesizing the oligonucleotide using the compound represented by Formula (I) or the pharmaceutically acceptable salt thereof.

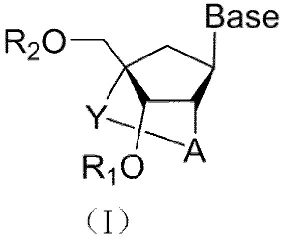

The preparation method of the oligonucleotide or pharmaceutically acceptable salt thereof provided in the present disclosure preferably include steps of synthesizing the oligonucleotide using at least one of the compounds represented below or the pharmaceutically acceptable salt thereof:

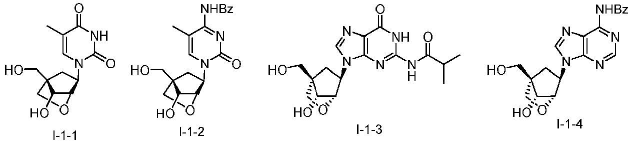

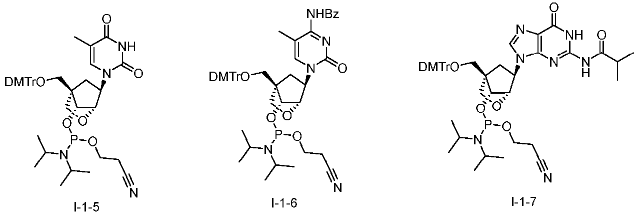

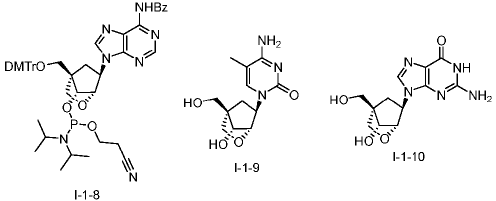

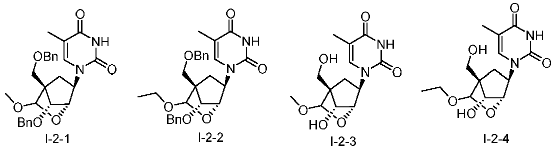

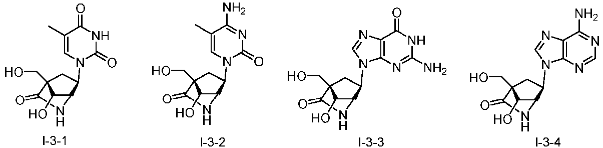

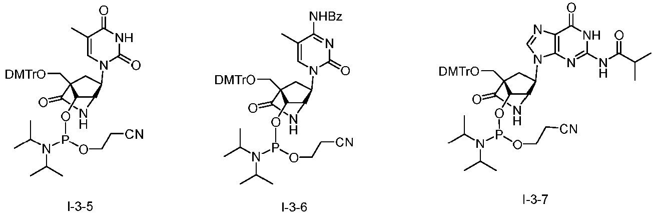

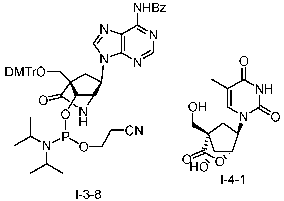

Further, the present disclosure provides a pharmaceutical composition including a therapeutically effective amount of one or more of the compounds or salt thereof, as well as a pharmaceutically acceptable excipient.

Further, the present disclosure provides medical uses of the compound in the preparation of a drug for gene therapy, gene vaccination, antisense therapy, interfering RNA, or nucleic acid transfer.

Further, the present disclosure provides a pharmaceutical composition including a therapeutically effective amount of one or more of the oligonucleotides or pharmaceutically acceptable salt thereof, as well as a pharmaceutically acceptable excipient.

Further, the present disclosure provides medical uses of the oligonucleotide or pharmaceutically acceptable salt thereof in the preparation of a drug for gene therapy, gene vaccination, antisense therapy, interfering RNA, or nucleic acid transfer.

Beneficial effects: Compared with prior art, the present disclosure has the following significant advantages: the conformationally locked carbon-ring nucleoside or nucleotide is provided according to the present disclosure, the oligonucleotide containing the conformationally locked carbon-ring nucleoside or nucleotide exhibits higher binding affinity to single-stranded RNA, nuclease resistance, and plasma stability, and is expected to be used in nucleic acid-based drugs.

### BRIEF DESCRIPTION OF THE DRAWINGS

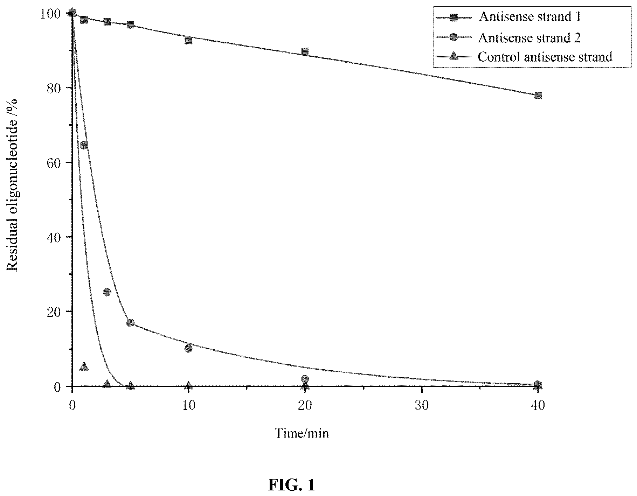

FIG. 1 is a statistical diagram of results of an anti-nuclease degradation experiment.

### DETAILED DESCRIPTIONS OF THE EMBODIMENTS

First of all, terms used in this specification are defined as follows.

In this specification, the term "C₁₋₄ alkyl group" refers to a straight-chain or branched alkyl group containing 1-4 carbon atoms, such as methyl, ethyl, n-propyl, n-butyl, isopropyl, isobutyl, tert-butyl, and the like. The term "C₃₋₇ cycloalkyl group" refers to a saturated or partially saturated monocyclic ring containing 3-7 carbon atoms, including cyclopropyl, cyclobutyl, cyclopentyl, cyclohexyl, cycloheptyl, and the like. The term "(C₁₋₄) alkyl-(C₃₋₇) cycloalkyl group" refers to a hydrocarbon compound containing 4-11 carbon atoms, in which a straight-chain or branched alkyl group containing 1-4 carbon atoms is connected to a saturated or partially saturated monocyclic ring containing 3-7 carbon atoms.

In this specification, the term "C₁₋₃ alkyl group" refers to a straight-chain or branched alkyl group containing 1-3 carbon atoms, and the alkyl includes methyl, ethyl, n-propyl, isopropyl, and the like. The term "C₁₋₃ alkyl-phenyl group" refers to a compound in which a straight-chain or branched alkyl group containing 1-3 carbon atoms is bonded to a phenyl group.

In this specification, the term "heterocycle" refers to a saturated or partially saturated monocyclic group containing 5-6 carbon atoms, in which one or more (1-3) carbon atoms are replaced by heteroatoms (such as O, N, or S). The term "C₁₋₃ alkyl-heterocycle" refers to a compound in which a straight-chain or branched alkyl group containing 1-3 carbon atoms is bonded to a heterocycle.

In this specification, the term "halogenoalkyl" refers to an alkyl group in which a hydrogen atom is replaced by a halogen atom (such as F, Cl, Br and I).

In this specification, the term "salt thereof" refers to salt of a compound represented by Formula I of the present disclosure, and preferably includes alkali metal salt such as sodium salt, potassium salt, and lithium salt; alkaline earth metal salt such as calcium salt, and magnesium salt; metal salt such as aluminum salt, iron salt, zinc salt, copper salt, nickel salt, and cobalt salt; amine salt of inorganic salt; amine salt of organic salt such as tert-octylamine salt, dibenzylamine salt, morpholine salt, glucosamine salt, phenylglycine alkyl ester salt, ethylenediamine salt, N-methylglucosamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenylethylamine salt, piperazine salt, tetramethylammonium salt, and tris(hydroxymethyl)aminomethane salt; halogenated hydrochloric acid salt such as hydrofluoric acid salt, hydrochloride salt, hydrobromic acid salt, hydroiodic acid salt; inorganic acid salt such as nitrate, perchlorate, sulfate, and phosphate; lower alkane sulfonate such as methanesulfonate salt, trifluoromethanesulfonate salt, ethylsulfonate salt; aryl sulfonate such as benzenesulfonate salt and p-toluenesulfonate salt; organic salt such as acetate, malate, fumarate, succinate, citrate, tartrate, oxalate, and maleate; and amino acid salt such as glycine salt, lysine salt, arginine salt, ornithine salt, glutamate salt, and aspartic acid salt.

In this specification, the term "pharmaceutically acceptable salt" refers to salt or zwitterion of a compound disclosed herein, which is water-soluble, oil-soluble, or dispersible, and is pharmaceutically acceptable. Salt can be prepared through the final separation and purification of the compound or by reacting an appropriate compound in the form of a free base with an appropriate acid. Acid that forms pharmaceutically acceptable salt can be inorganic acid such as hydrochloric acid, hydrobromic acid, sulfuric acid, and phosphoric acid, or organic acid such as oxalic acid, maleic acid, succinic acid, and citric acid. Salt can also be formed through coordination of a compound with alkali metal or alkaline earth ions (such as sodium), alkaline earth metal (such as magnesium and calcium), ammonium and NX4⁺ ions (where X is a C1-C4 alkyl group). For therapeutic use, active pharmaceutical ingredient compound salt in the present disclosure is generally physiologically acceptable, that is, they are derived from physiologically acceptable acid or base. However, salt of physiologically unacceptable acid or base can also be used for preparing or purifying the physiologically acceptable compounds. All salt, whether or not derived from physiologically acceptable acid or base, fall within the scope of the present disclosure.

In this specification, the term "pharmaceutically acceptable excipient" refers to an additive other than an active ingredient in a pharmaceutical formulation, which does not cause significant side effects when administered. The pharmaceutically acceptable excipient includes but is not limited to solvent, co-solvent, preservative, antioxidant, solubilizer, emulsifier, and osmoregulatory agent in liquid preparations, as well as adhesive, filler, disintegrant, and lubricant in preparations.

The conformationally locked carbon-ring nucleoside or nucleotide of the present disclosure can be present at any position of a nucleic acid drug, and a number can be one or more. The position and number thereof are not particularly limited and can be designed according to specific purpose. Nucleic acid drugs are a type of drugs that consist of a plurality of nucleotides and specifically act on target nucleic acid in the form of complementary base pairing, thereby regulating gene expression to achieve disease treatment. The nucleic acid drug include but are not limited to ASO, siRNA, miRNA, saRNA, Aptamer, Ribozymes, mRNA, ARC, Plasmid DNA, CRISPR/Cas9, and the like. Specifically, ASO is usually a single-stranded oligonucleotide composed of 12-30 nucleotides, forms a double-stranded structure target mRNA after entering a cell, and regulates RNA function through different mechanisms. siRNA is a linear double-stranded RNA with a length of 21-25 nucleotides, including one sense strand and one antisense strand, and binds to an RNA-induced silencing complex (RISC) in the cytoplasm, and fully pairs with a target mRNA, and the target gene is silenced by mRNA degradation. miRNA is a non-coding RNA with a length of 19-24 nucleotides. Mature miRNA is a single-stranded RNA. After binding to mRNA in a cell, miRNA performs post-transcriptional regulation, inhibits protein translation and regulates the expression of target genes. saRNA is a short double-stranded oligonucleotide with a structure similar to that of siRNA, it can increase mRNA expression and upregulate target protein by recruiting an endogenous transcription complex to a target gene, thus exerting therapeutic effects. Aptamer is single-stranded nucleic acid with a stable secondary structure and a length of 25-50 nucleotides, which can specifically bind to target molecules such as small molecules, protein, bacteria, and viruses with the same length. Experienced professionals can use general nucleic acid synthesis methods, which are not limited to those described in the examples, to synthesize nucleotide analogs containing the conformationally locked carbon ring structure of the present disclosure, and then further synthesize various nucleic acid drugs.

When forming a double-stranded structure with complementary nucleic acid, nucleic acid containing the conformationally locked carbon ring structure of the present disclosure is easy to form a stable double-stranded chain with the target nucleic acid chain because the ribose analog containing the carbocyclic ring is partially locked, the transcription and translation process is inhibited, affecting pathogen protein synthesis is affected, proliferation of infectious viruses is inhibited, high binding affinity and nuclease resistance are implemented, and residence time in the body is prolonged.

Preferably, the nucleic acid containing the conformationally locked carbon ring structure of the present disclosure is preferably administered to a living body in the form of pharmaceutical preparation, and can be prepared into an injection preparation together with excipients such as preservatives, antioxidants, and other adjuvants used in the field of pharmaceutical preparation technology. In addition, the nucleic acid containing the conformationally locked carbon ring structure of the present disclosure can also be formulated with pharmaceutical excipients used in the technical field into liquid preparations, solid preparations, ointments, and the like, and administered through gastrointestinal tract or skin.

The nucleic acid containing the conformationally locked carbon ring structure of the present disclosure can be expected to be used to treat or prevent certain diseases by inhibiting the gene expression, such as antiviral or anticancer therapies.

The compound in the present disclosure is a compound or salt represented by the following Formula (I): 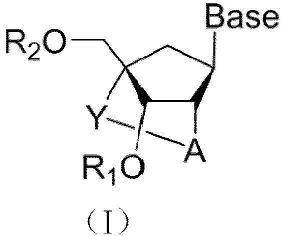
where R₁ and R₂ independently represent a hydrogen atom, an alkyl group containing 1-6 carbon atoms, a phenyl group, a (C₁₋₃) alkyl-phenyl group, a heterocycle, or a (C₁₋₃) alkyl-heterocycle, a hydroxyl protecting group, a silicon group containing one or more substituents, a phosphate group containing a protecting group for oligonucleotide synthesis, phosphoester, thiophosphate, chiral thiophosphate, phosphate triester, aminoalkyl phosphate triester, and alkyl phosphate ester;
A is O, S, or NR₃; R₃ is a hydrogen atom, an alkyl group containing 1-6 carbon atoms, a halogenated alkyl group containing 1-6 carbon atoms, -C(O)R₄, -C(O)OR₄, or -C(O)N(R₄)₂; and R₄ is H, or an alkyl group containing 1-6 carbon atoms or a halogenated alkyl group containing 1-6 carbon atoms;
Y is C=O, CHR₅, or CHOR₅; and R₅ is H, or an alkyl group containing 1-6 carbon atoms or a halogenated alkyl group containing 1-6 carbon atoms; and
Base is a base.

Preferably, Formula (I) is represented by any one of the following Formulae (I-1) - (I-4):

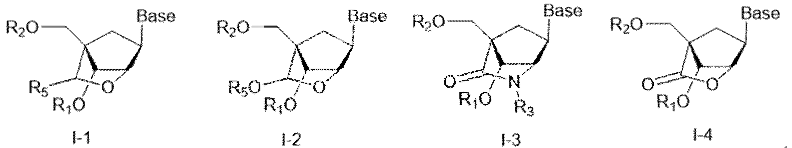

Preferably, the compound represented by Formula (I) has any of the structures shown in the following formula:

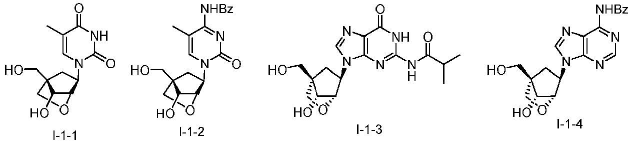

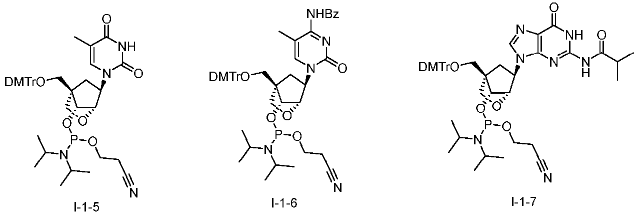

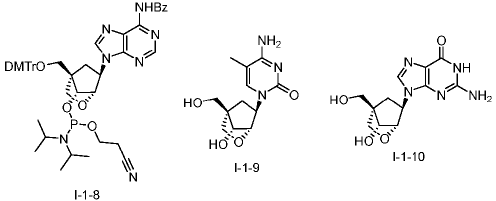

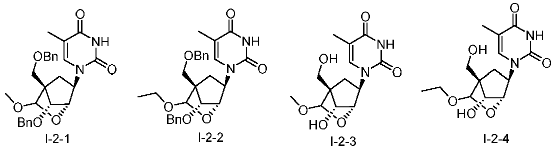

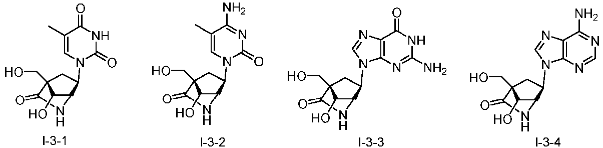

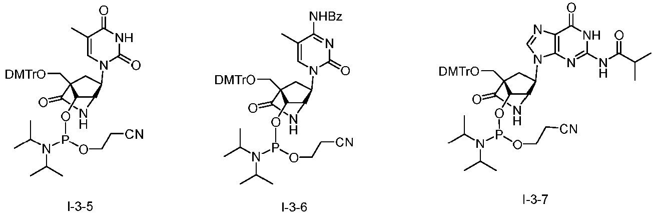

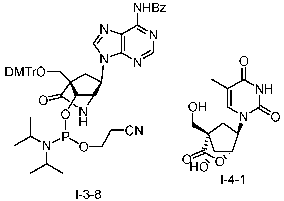

Further, the present disclosure provides an oligonucleotide or pharmaceutically acceptable salt thereof, which contains at least one of the structures shown in Formulae (II)-(IV): 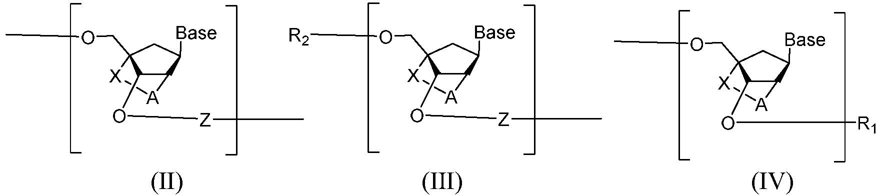 where Z is phosphoester, thiophosphate, chiral thiophosphate, phosphate triester, aminoalkyl phosphate triester, and alkyl phosphate ester.

### Examples

Synthesis of the conformationally locked carbon-ring nucleoside and analogs thereof in the present disclosure is described in detail based on the examples below.

### Example 1: Synthesis of Compound I-1-1

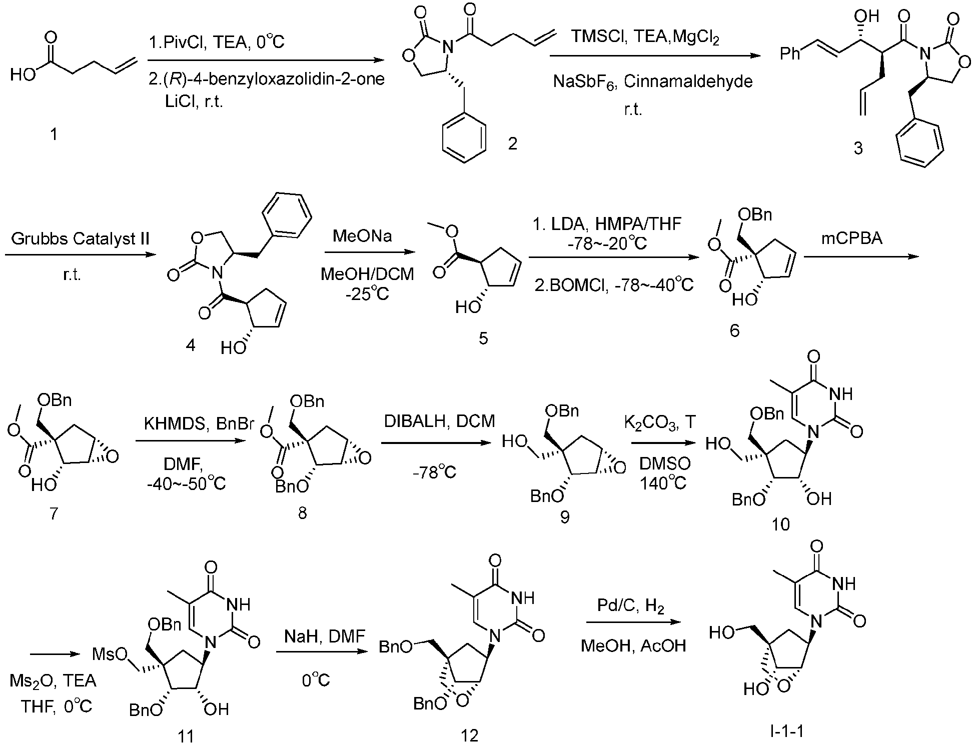

### (1) Synthesis of Compound 5

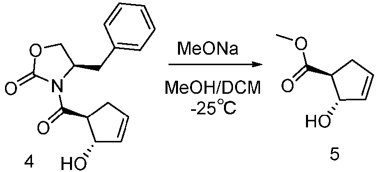

Under a nitrogen atmosphere, Compound 4 (575 g, 2 mol) (reference for synthesis: Journal of Medicinal Chemistry, 2014, Vol. 57, #5, p. 2107-2120) was dissolved in 10 L of dichloromethane to obtain a mixture, a temperature was lowered to -20 to -25°C, 5 L of 0.4M sodium methoxide in methanol solution was added dropwise, and the mixture was stirred for 30 min after the addition was completed. The reaction was monitored by thin-layer chromatography to ensure the reaction was completed, 4M of HCl 1,4-dioxane was added to quench the reaction after the reaction was completed, and a pH was adjusted to <7. Filtering and concentration were performed to obtain a crude product, the crude product was purified by column chromatography with an eluent of petroleum ether: ethyl acetate =5:1- 3:1, and 228 g of an oily substance was obtained, with a yield of 80%. [M+1]⁺=143.1; 1H NMR (400 MHz, Chloroform-d) δ 5.98 - 5.87 (m, 1H), 5.76 (dq, J = 5.9, 2.1 Hz, 1H), 5.12 (dp, J = 5.6, 1.8 Hz, 1H), 3.75 (s, 3H), 2.96 (ddd, J = 9.0, 6.7, 5.2 Hz, 1H), 2.78 (ddq, J = 15.9, 9.0, 2.3 Hz, 1H), 2.66 - 2.55 (m, 1H).

### (2) Synthesis of Compound 6

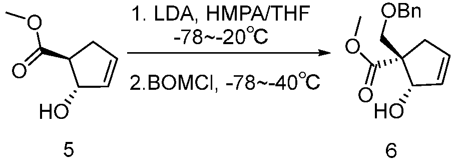

Under a nitrogen atmosphere, Compound 5 (50 g, 0.352 mol) was dissolved in 500 ml of dry tetrahydrofuran and 170 ml of hexamethylphosphoric triamide to obtain a mixture, a temperature was lowered to -78°C, 530 ml of tetrahydrofuran solution of 2M lithium diisopropylamide solution was added dropwise, a temperature of the mixture was raised to -20°C and stirred for 30-45 min after the addition was completed, the temperature was then lowered to -78°C again, a dry tetrahydrofuran solution of benzylchloromethyl ether (66 g, 0.422 mol) was added dropwise, and the temperature was then slowly raised up to -40°C after the addition was completed and allowed to react for 2 h. The reaction was monitored by gas chromatography to ensure the reaction was completed, acetic acid was added to quench the reaction, a temperature was raised to room temperature, saturated ammonium chloride solution was added, extraction was performed twice with ethyl acetate, organic phases were combined and washed with saturated brine in turn, and dried with anhydrous sodium sulfate, filtering and concentration were performed to obtain a crude product, the crude product was purified by the column chromatography with an eluent of petroleum ether: ethyl acetate =5:1- 4:1, and 63 g of an oily substance was obtained, with a yield of 68%. [M+1]⁺=263.2; 1H NMR (400 MHz, Chloroform-d) δ 7.40 - 7.29 (m, 5H), 5.99 (dt, J = 5.3, 2.3 Hz, 1H), 5.76 (dq, J = 6.8, 2.2 Hz, 1H), 4.57 (d, J = 2.3 Hz, 1H), 4.54 (s, 2H), 3.79 (d, J = 8.7 Hz, 1H), 3.77 (s, 3H), 3.39 (d, J = 8.7 Hz, 1H), 3.12 (dt, J = 17.8, 2.4 Hz, 1H), 2.55 (dt, J = 17.8, 2.3 Hz, 1H).

### (3) Synthesis of Compound 7

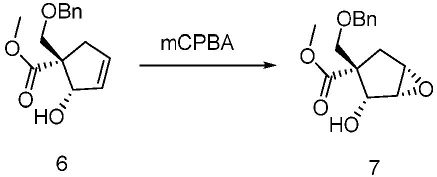

Under a nitrogen atmosphere, Compound 6 (63 g, 0.24 mol) was dissolved in 600 ml of dichloromethane to obtain a mixture, a temperature was lowered to 0°C, and 85% meta-chloroperoxybenzoic acid (121.8 g, 0.6 mol) was added in batches, and stirred at room temperature overnight. The reaction was monitored by a liquid chromatography-mass spectrometry (LCMS) to ensure the reaction was completed, saturated sodium sulfite solution was added to quench, organic phases were then washed with saturated sodium bicarbonate solution in turn, and dried with anhydrous sodium sulfate, filtering and concentration were performed to obtain a crude product, the crude product was purified by the column chromatography with an eluent of petroleum ether: ethyl acetate =5:1- 4:1, and 45g of an oily substance was obtained, with a yield of 67%. [M+1]⁺=288.1.

### (4) Synthesis of Compound 8

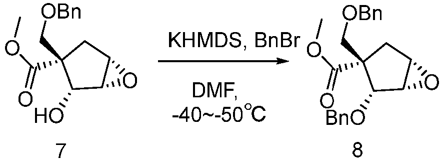

Under a nitrogen atmosphere, Compound 7 (35.2 g, 0.126 mol) was dissolved in 250 ml of dry N,N-dimethylformamide, benzyl bromide (32.3 g, 0.189 mol) was then added to obtain a mixture, a temperature was lowered to -40 to -50°C, 189 ml of tetrahydrofuran solution of 1M potassium bis(trimethylsilyl)amide was added dropwise, and the reaction was continued for 1 h after the addition was completed. The reaction was monitored by the LCMS to ensure the reaction was completed, acetic acid was added to quench the reaction, a temperature was raised to room temperature, saturated ammonium chloride solution was added, extraction was performed twice with ethyl acetate, organic phases were combined and washed with saturated brine twice in turn, and dried with anhydrous sodium sulfate, filtering and concentration were performed to obtain a crude product, the crude product was purified by the column chromatography with an eluent of petroleum ether: ethyl acetate =5:1- 4:1, and 40g of an oily substance was obtained, with a yield of 84%. [M+1]⁺=369.2.

### (5) Synthesis of Compound 9

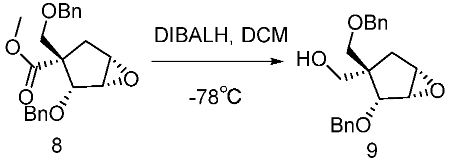

Under a nitrogen atmosphere, Compound 8 (39 g, 0.106 mol) was dissolved in 1.2 L of dry dichloromethane to obtain a mixture, a temperature was lowered to -78°C, 254 ml of n-hexane solution of 1M diisobutylaluminium hydride was added dropwise, and the reaction was continued for 1 h at -78°C after the addition was completed. The reaction was monitored by the LCMS to ensure the reaction was completed, acetic acid was added to quench the reaction, a temperature was raised to room temperature, organic phases were then washed with saturated aqueous sodium bicarbonate solution in turn, and dried with saturated brine twice, filtering and concentration were performed to obtain a crude product, the crude product was purified by the column chromatography with an eluent of petroleum ether: ethyl acetate =4:1, and 28 g of an oily substance was obtained, with a yield of 77.7%. [M+1]⁺=341.2; 1H NMR (400 MHz, Chloroform-d) δ 7.41 - 7.29 (m, 7H), 4.80 (d, J = 11.9 Hz, 1H), 4.60 (d, J = 11.9 Hz, 1H), 4.47 (q, J = 12.1 Hz, 2H), 4.20 (s, 1H), 3.87 (d, J = 11.2 Hz, 1H), 3.60 (d, J = 2.7 Hz, 1H), 3.47 (d, J = 8.3 Hz, 3H), 3.37 (d, J = 9.1 Hz, 1H), 2.98 (s, 1H), 2.05 - 1.99 (m, 1H), 1.92 (d, J = 14.9 Hz, 1H).

### (6) Synthesis of Compound 10

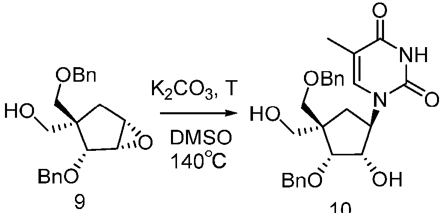

Under a nitrogen atmosphere, Compound 9 (22 g, 0.065 mol), thymine (32.8 g, 0.26 mol) and potassium carbonate (26.9 g, 0.195 mol) were suspended in 220 ml of dry dimethyl sulfoxide, heated to 140 °C and stirred for 3 h to obtain a mixture. The reaction was monitored by the LCMS to ensure the reaction was completed, a temperature was lowered to room temperature, water was added, extraction was performed twice with ethyl acetate, organic phases were combined and washed with saturated brine twice in turn, and dried with anhydrous sodium sulfate, filtering and concentration were performed to obtain a crude product, the crude product was purified by the column chromatography with an eluent of dichloromethane: methanol (50:1-20:1), and 24.4 g of a white solid substance was obtained, with a yield of 81%. [M+1]⁺=467.2; 1H NMR (400 MHz, DMSO-d6) δ 11.19 (s, 1H), 7.55 - 7.16 (m, 11H), 5.09 (d, J = 6.5 Hz, 1H), 4.81 (d, J = 11.7 Hz, 1H), 4.71 (q, J = 9.7 Hz, 1H), 4.59 - 4.45 (m, 4H), 4.39 (dt, J = 9.7, 5.8 Hz, 1H), 3.80 (d, J = 4.9 Hz, 1H), 3.61 - 3.39 (m, 4H), 1.93 - 1.82 (m, 1H), 1.68 (d, J = 1.1 Hz, 3H), 1.35 (dd, J = 13.5, 10.1 Hz, 1H).

### (7) Synthesis of Compound 11

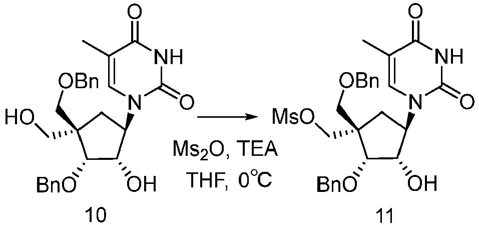

Under a nitrogen atmosphere, Compound 10 (17.3 g, 0.037 mol) and triethylamine (15 g, 0.148 mol) were dissolved in 170 ml of dry tetrahydrofuran to obtain a mixture, a temperature was lowered to -10 °C, tetrahydrofuran solution of methylsulfonic anhydride (7.7 g, 0.044 mol) was added dropwise, and the reaction was continued at -10 °C for 30 min after the addition was completed. The reaction was monitored by the LCMS to ensure the reaction was completed, saturated aqueous sodium bicarbonate solution was added, extraction was performed twice with ethyl acetate, organic phases were combined and washed with saturated brine twice in turn, and dried with anhydrous sodium sulfate, filtering and concentration were performed to obtain a crude product, the crude product was purified by the column chromatography with an eluent of dichloromethane: methanol =100:1, and 14.5 g of a white solid substance was obtained, with a yield of 72.5%. [M+1]⁺=545.2.

### (8) Synthesis of Compound 12

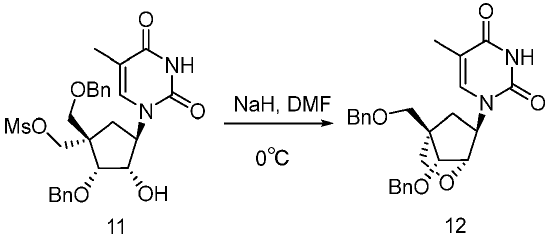

Under a nitrogen atmosphere, 60% sodium hydride (3.2 g, 0.081 mol) was carefully added to 100 ml of dry N, N-dimethylformamide to obtain a mixture, a temperature was lowered to 0°C, Compound 11 (14 g, 0.027 mol) was added dropwise in a N, N-dimethylformamide solution, and the reaction was continued for 1 h after the addition was completed. The reaction was monitored by the LCMS to ensure the reaction was completed, saturated ammonium chloride solution was added, extraction was performed twice with ethyl acetate, organic phases were combined and washed with saturated sodium chloride twice in turn, and dried with anhydrous sodium sulfate, filtering and concentration were performed to obtain a crude product, the crude product was purified by the column chromatography with an eluent of dichloromethane: methanol =40:1, and 8.5 g of oily liquid was obtained, with a yield of 74%. [M+1]⁺=449.2.

### (9) Synthesis of Compound I-1-1

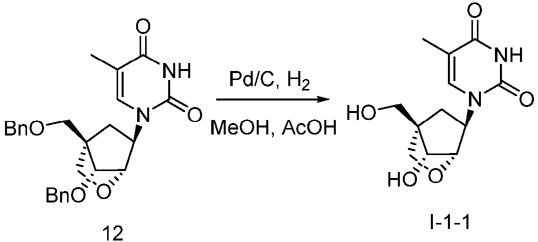

Compound 12 (4 g, 8.9 mmol) was dissolved in 40 ml of anhydrous methanol, 50 µl of acetic acid and 400 mg of 10% palladium carbon (60% water content) were added to obtain a mixture, hydrogen was purged three times, and the mixture was stirred at room temperature overnight. The reaction was monitored by the LCMS to ensure the reaction was completed, and filtering and concentration were performed to obtain 2.2 g of white solid, with a yield of 90%. [M+1]⁺=269.2; 1H NMR (400 MHz, DMSO-d6) δ 11.27 (s, 1H), 7.43 (d, J = 1.3 Hz, 1H), 5.76 (s, 2H), 4.23 (dd, J = 9.7, 5.6 Hz, 1H), 3.91 (d, J = 14.6 Hz, 2H), 3.70 - 3.61 (m, 2H), 3.53 - 3.43 (m, 3H), 2.05 (dd, J = 13.6, 9.7 Hz, 1H), 1.81 - 1.78 (m, 3H), 1.78 - 1.72 (m, 1H).

### Example 2: Synthesis of Compound I-1-2

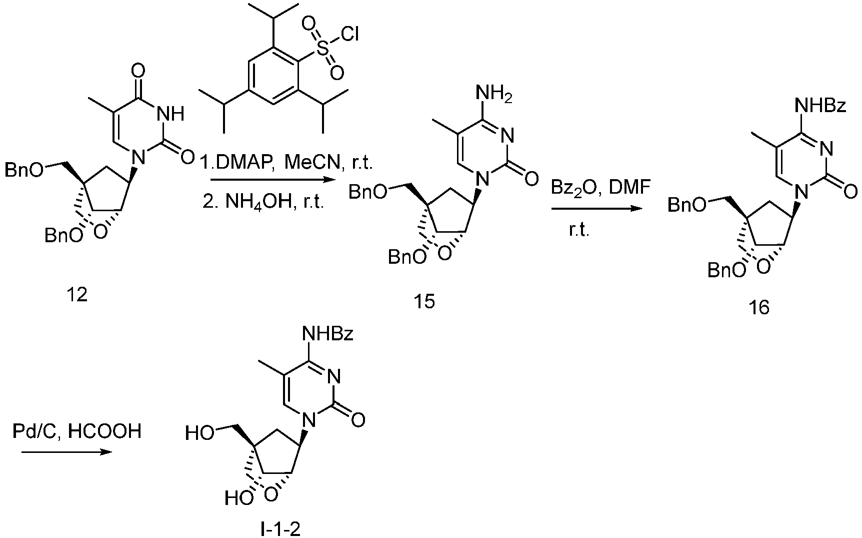

### (1) Synthesis of Compound 15

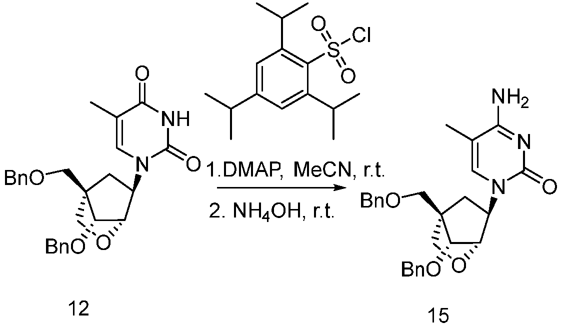

Under a nitrogen atmosphere, Compound 12 (8.5 g, 0.019 mol), 4-dimethylaminopyridine (4.6 g, 0.038 mol), and triethylamine (7.7 g, 0.076 mol) were dissolved in 100 ml of dry acetonitrile, and 2,4,6-triisopropylbenzenesulfonyl chloride (11.5 g, 0.038 mol) was carefully added to obtain a mixture, the mixture was stirred at room temperature for 2 h, and cooled down to 0°C, 28.5 ml of concentrated ammonia water was added, and stirred overnight after addition was completed. The reaction was monitored by the LCMS to ensure the reaction was completed, extraction was performed twice with ethyl acetate, and organic phases were combined and washed with saturated ammonium chloride and saturated brine in turn, and dried with anhydrous sodium sulfate, filtering and concentration were performed to obtain a crude product, the crude product was purified by the column chromatography with an eluent of dichloromethane: methanol =30:1, and 6g of oily liquid was obtained, with a yield of 70.7%. [M+1]⁺=448.2.

### (2) Synthesis of Compound 16

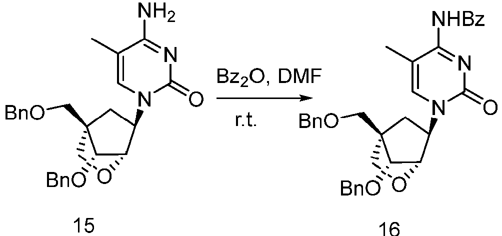

Under a nitrogen atmosphere, Compound 15 (4.6 g, 0.010 mol) was dissolved in 35 ml of dry N,N-dimethylformamide, and benzoic anhydride (3.2 g, 0.014 mol) was added and stirred at room temperature overnight. The reaction was monitored by the LCMS to ensure the reaction was completed, saturated sodium bicarbonate solution was added, extraction was performed twice with ethyl acetate, organic phases were combined and washed with saturated brine in turn, and dried with anhydrous sodium sulfate, filtering and concentration were performed to obtain a crude product, the crude product was purified by the column chromatography with an eluent of petroleum ether: ethyl acetate =3:1, and 3.8g of oily liquid was obtained, with a yield of 68.9%. [M+1]⁺=552.2.

### (3) Synthesis of Compound I-1-2

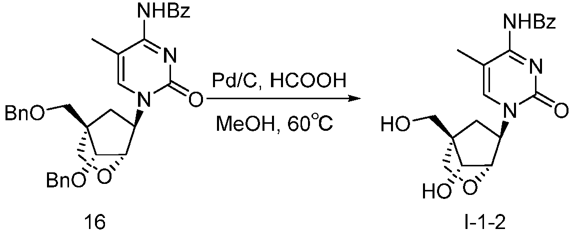

Under a nitrogen atmosphere, Compound 16 (2 g, 3.6 mmol) was dissolved in 20 ml of anhydrous methanol, 4 ml of formic acid and 960 mg of 10% palladium carbon (60% water content) were added, and stirred at 60 °C for 6 h. The reaction was monitored by the LCMS to ensure the reaction was completed, and filtering, washing and concentration were performed to obtain 1.1 g of white solid, with a yield of 85%. [M+1]⁺=372.2; 1H NMR (400 MHz, DMSO-d6) δ 13.17 (s, 1H), 8.21 (s, 2H), 7.79 (s, 1H), 7.60 (t, J = 7.3 Hz, 1H), 7.50 (t, J = 7.6 Hz, 2H), 5.13 (d, J = 4.5 Hz, 1H), 4.56 (t, J = 5.3 Hz, 1H), 4.31 (dd, J = 9.7, 5.7 Hz, 1H), 4.06 (s, 1H), 3.93 (d, J = 4.5 Hz, 1H), 3.75 - 3.61 (m, 2H), 3.52 (dd, J = 15.8, 5.9 Hz, 2H), 2.19 - 2.09 (m, 1H), 2.06 (s, 3H), 1.88 (d, J = 13.1 Hz, 1H).

### Example 3: Synthesis of Compound I-1-3

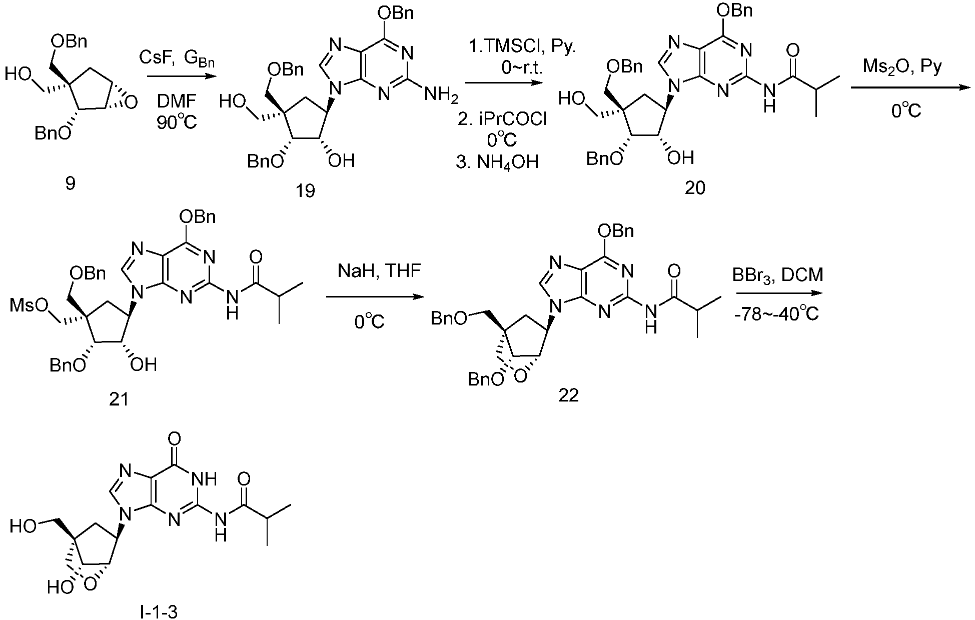

### (1) Synthesis of Compound 19

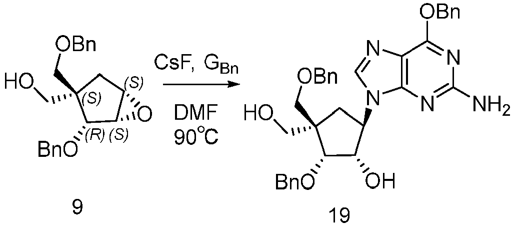

Under a nitrogen atmosphere, Compound 9 (24 g, 0.07 mol), cesium fluoride (31.9 g, 0.21 mol) and O-6-Benzylguanine (33.8 g, 0.14 mol) were suspended in 240 ml of dry DMF and heated to 90 °C for 3 h; The reaction was monitored by the LCMS to ensure the reaction was completed, a temperature was lowered, water was added, extraction was performed twice with ethyl acetate, organic phases were combined and washed with saturated brine twice in turn, and dried with anhydrous sodium sulfate, filtering and concentration were performed to obtain a crude product, the crude product was purified by the column chromatography with an eluent of petroleum ether: ethyl acetate =1:2- 1:3, and 23.3g of an oily substance was obtained, with a yield of 56.8%. [M+1]⁺=582.2; 1H NMR (400 MHz, Chloroform-d) δ 7.64 (s, 1H), 7.57 - 7.47 (m, 2H), 7.46 - 7.29 (m, 13H), 5.58 (s, 2H), 4.94 (d, J = 11.4 Hz, 1H), 4.88 (s, 2H), 4.70 (dd, J = 10.8, 3.0 Hz, 2H), 4.57 (dd, J = 8.4, 5.6 Hz, 1H), 4.52 (s, 2H), 4.15 (d, J = 5.6 Hz, 1H), 3.85 (d, J = 11.3 Hz, 1H), 3.66 (d, J = 11.3 Hz, 1H), 3.41 (s, 2H), 2.38 (dd, J = 13.3, 8.7 Hz, 1H), 2.09 - 1.96 (m, 2H).

### (2) Synthesis of Compound 20

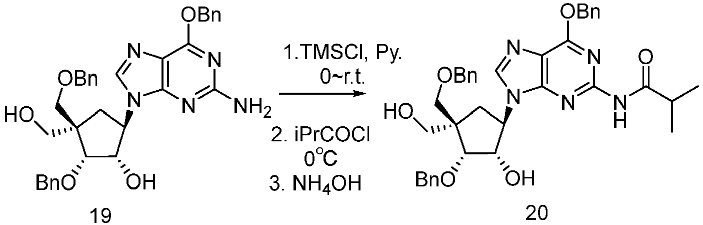

Under a nitrogen atmosphere, Compound 19 (23.2 g, 0.04 mol) was dissolved in 230 ml of dry pyridine to obtain a mixture, a temperature was lowered to 0 °C, trimethylchlorosilane (26.1 g, 0.24 mol) was added dropwise, and stirred at room temperature for 2 h after the addition was completed, and then cooled down to 0°C, isobutyryl chloride (4.7 g, 0.044 mol) was added dropwise and stirred for 2 h after the addition was completed; and 30 ml of concentrated ammonia was added dropwise and stirred at room temperature for 1 h. The reaction was monitored by the LCMS to ensure the reaction was completed, water was added, extraction was performed twice with ethyl acetate, organic phases were combined and washed with saturated brine in turn, and dried with anhydrous sodium sulfate, filtering and concentration were performed to obtain a crude product, the crude product was purified by the column chromatography with an eluent of petroleum ether: ethyl acetate =1:1- 1:2, and 26.3 g of oily liquid was obtained, with a yield of 97%. [M+1]⁺=652.3.

### (3) Synthesis of Compound 21

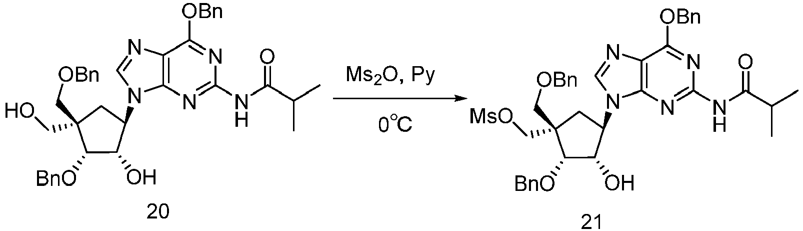

Under a nitrogen atmosphere, Compound 20 (25.5 g, 0.039 mol) were dissolved in 200 ml of dry tetrahydrofuran to obtain a mixture, a temperature was lowered to 0°C, tetrahydrofuran solution of methylsulfonic anhydride (8.7 g , 0.051 mol) was added dropwise, and stirred for 2 h after the addition was completed. The reaction was monitored by the LCMS to ensure the reaction was completed until a product content reached a maximum, water was added to quench, extraction was performed twice with ethyl acetate, organic phases were combined and washed with saturated brine in turn, and dried with anhydrous sodium sulfate, filtering and concentration were performed to obtain a crude product, the crude product was purified by the column chromatography with an eluent of petroleum ether: ethyl acetate =1:1, and 19.1g of an oily substance was obtained, with a yield of 56.8%. 4.5 g of raw material was recovered. [M+1]⁺=730.3.

### (4) Synthesis of Compound 22

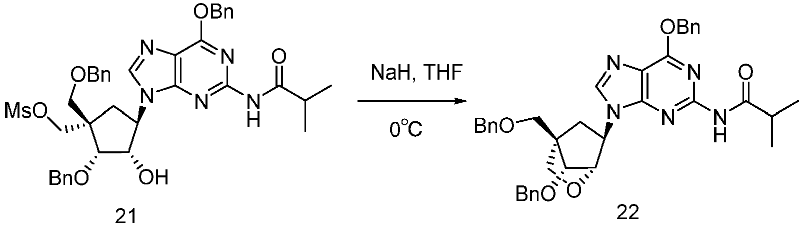

Under a nitrogen atmosphere, 60% sodium hydride (3.5 g, 87.9 mmol) was carefully added to 210 ml of dry tetrahydrofuran and stirred for 10 min to obtain a mixture, a temperature was lowered to 0 °C, and tetrahydrofuran solution of Compound 21 (21.4 g, 29.3 mmol) was added dropwise and stirred for 2 h after the addition was completed. The reaction was monitored by the LCMS to ensure the reaction was completed and quenched with acetic acid, saturated sodium bicarbonate solution was added, extraction was performed with ethyl acetate, organic phases were combined and washed with saturated brine in turn, and dried with anhydrous sodium sulfate, filtering and concentration were performed to obtain a crude product, the crude product was purified by the column chromatography with an eluent of petroleum ether: ethyl acetate =1:1, and 15.8 g of an oily substance was obtained, with a yield of 85%. [M+1]⁺=634.3.

### (5) Synthesis of Compound I-1-3

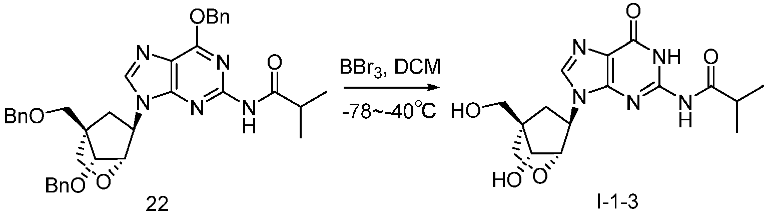

Under a nitrogen atmosphere, Compound 22 (5 g, 7.9 mmol) was dissolved in 50 ml of dichloromethane to obtain a mixture, a temperature was lowered to -78°C, and 71 ml of a dichloromethane solution of 1M boron tribromide was added dropwise, and a temperature was slowly raised to -40°C and stirred for 2 h after the addition was completed. The reaction was monitored by the LCMS to ensure the reaction was completed, methanol was added dropwise to quench, the temperature was raised to room temperature, and the mixture was concentrated to obtain a crude product, the crude product was purified by the column chromatography with an eluent of dichloromethane: methanol =10:1, and 2.5 g of a white solid was obtained, with a yield of 87.2%. [M+1]⁺= 364.1; 1H NMR (400 MHz, DMSO-d6) δ 8.14 (s, 1H), 7.36 - 7.21 (m, 1H), 5.17 (d, J = 4.2 Hz, 1H), 4.59 (t, J = 5.2 Hz, 1H), 4.44 (dd, J = 9.7, 5.4 Hz, 1H), 4.03 (d, J = 4.4 Hz, 1H), 3.96 (s, 1H), 3.78 - 3.68 (m, 2H), 2.79 (p, J = 6.8 Hz, 1H), 2.23 (ddd, J = 26.1, 14.8, 10.2 Hz, 2H), 1.99 (dd, J = 10.2, 6.6 Hz, 1H), 1.12 (d, J = 6.8 Hz, 5H).

### Example 4: Synthesis of Compound I-1-4

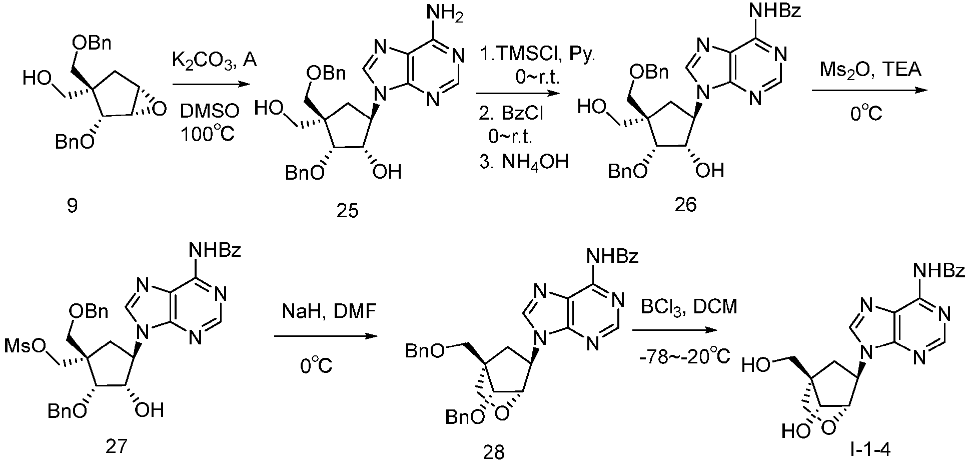

### (1) Synthesis of Compound 25

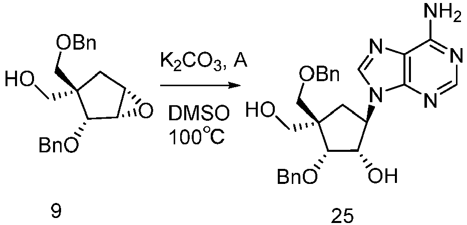

Under a nitrogen atmosphere, Compound 9 (26 g, 0.076 mol), adenine (20.5 g, 0.15 mol) and potassium carbonate (31.5 g, 0.23 mol) were suspended in 260 ml of dry dimethyl sulfoxide, heated to 120 °C and reacted for 4 h to obtain a mixture. The reaction was monitored by the LCMS to ensure the reaction was completed, a temperature was lowered, water was added, extraction was performed twice with ethyl acetate, organic phases were combined and washed with saturated brine in turn, and dried with anhydrous sodium sulfate, to obtain a crude product, the crude product was purified by the column chromatography with an eluent of dichloromethane: methanol = 20:1, and 29.2 g of a white solid was obtained, with a yield of 80.4%. [M+1]⁺=476.2; 1H NMR (400 MHz, DMSO-d6) δ 8.14 (s, 1H), 8.10 (s, 1H), 7.43 - 7.24 (m, 10H), 7.16 (s, 2H), 5.19 (d, J = 6.2 Hz, 1H), 4.84 (d, J = 11.8 Hz, 1H), 4.81 - 4.71 (m, 2H), 4.61 - 4.53 (m, 3H), 4.50 (t, J = 4.9 Hz, 1H), 3.90 (d, J = 4.3 Hz, 1H), 3.65 - 3.56 (m, 2H), 3.54 (d, J = 4.6 Hz, 2H), 2.10 (dd, J = 13.5, 8.6 Hz, 1H), 1.82 (dd, J = 13.4, 9.2 Hz, 1H).

### (2) Synthesis of Compound 26

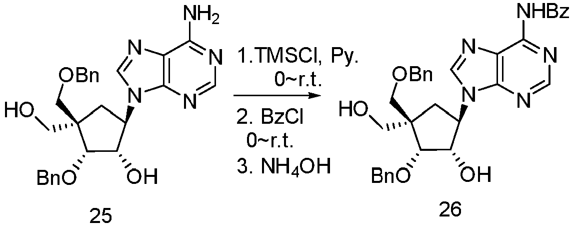

Under a nitrogen atmosphere, Compound 25 (22 g, 0.046 mol) was dissolved in 200 ml of dry pyridine to obtain a mixture, a temperature was lowered to 0 °C, trimethylchlorosilane (25 g, 0.231 mol) was added dropwise, and stirred at room temperature for 2 h after the addition was completed, a temperature was lowered to 0°C, benzoyl chloride (13.5 g, 0.097 mol) was added dropwise, and then stirred at room temperature for 2 h after the addition was completed. The reaction was monitored by the LCMS to ensure the reaction was completed, a temperature was raised to 0°C, 35 ml of concentrated ammonia water was added, a temperature was raised to room temperature, and the mixture was stirred for 1 h. The reaction was monitored by the LCMS to ensure the reaction was completed, water was added, extraction was performed twice with ethyl acetate, organic phases were combined and washed with saturated brine in turn, and dried with anhydrous sodium sulfate, filtering and concentration were performed to obtain a crude product, the crude product was purified by the column chromatography with an eluent of 100% ethyl acetate, and 20 g of substance was obtained, with a yield of 73.7%. [M+1]⁺=560.3.

### (3) Synthesis of Compound 27

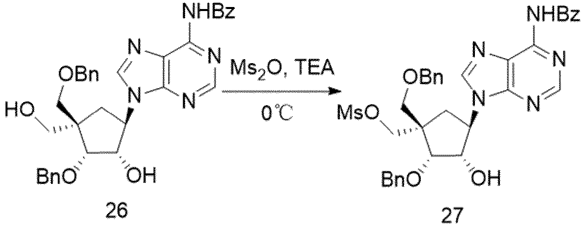

Under a nitrogen atmosphere, Compound 26 (20 g, 0.034 mol) and methylsulfonic anhydride (7.2 g, 0.041 mol) were dissolved in 200 ml of dry tetrahydrofuran to obtain a mixture, a temperature was lowered to -10°C, triethylamine (10.4 g, 0.103 mol) was added dropwise, a temperature was raised to 0 °C after the addition was completed, and the reaction was continued for 2 h. The reaction of raw material was monitored by the LCMS to ensure that the reaction was basically completed, saturated aqueous sodium bicarbonate solution was added, extraction was performed with ethyl acetate, organic phases were combined and washed with saturated brine in turn, and dried with anhydrous sodium sulfate, filtering and concentration were performed to obtain a crude product, the crude product was purified by the column chromatography with an eluent of petroleum ether: ethyl acetate =1:1, and 17 g of a white solid was obtained, with a yield of 65%. [M+1]⁺=658.2.

### (4) Synthesis of Compound 28

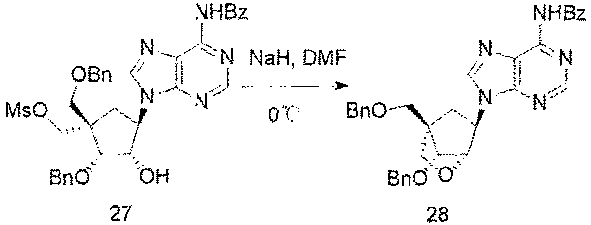

Under a nitrogen atmosphere, 60% of sodium hydride (1.87 g, 77.7 mmol) was carefully added to 170 ml of dry N, N-dimethylformamide to obtain a mixture, the mixture was stirred 10 min and cooled down to 0°C, Compound 27 (17 g, 25.9 mmol) was added dropwise in a N, N-dimethylformamide solution and continuously stirred for 2 h after the addition was completed. The reaction was monitored by the LCMS to ensure the reaction was completed, saturated ammonium chloride solution was added, extraction was performed twice with ethyl acetate, organic phases were combined and washed with saturated brine in turn, and dried with anhydrous sodium sulfate, filtering and concentration were performed to obtain a crude product, the crude product was purified by the column chromatography with an eluent of 100% ethyl acetate, and 12.3 g of a white solid was obtained, with a yield of 85%. [M+1]⁺=562.2.

### (5) Synthesis of Compound I-1-4

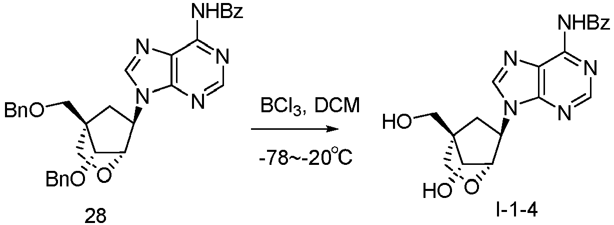

Under a nitrogen atmosphere, Compound 28 (5.3 g, 10.7 mmol) was dissolved in 50 ml of dry dichloromethane to obtain a mixture, a temperature was lowered to -78°C, and 64 ml of a dichloromethane solution of 1M boron trichloride was added dropwise, and a temperature was slowly raised to -20°C and stirred overnight after the addition was completed. The reaction was monitored by the LCMS to ensure the reaction was completed. After concentration under a reduced pressure was performed, ethyl acetate was added to obtain a mixture, the mixture was filtered to obtain 3.4 g of a white solid, which was the hydrochloride salt form of the compound in Example 4. [M+1]⁺=382.2; 1H NMR (400 MHz, DMSO-d6) δ 11.16 (s, 1H), 8.71 (s, 1H), 8.55 (s, 1H), 8.11 - 8.02 (m, 2H), 7.71 - 7.63 (m, 1H), 7.56 (dd, J = 8.3, 7.0 Hz, 2H), 7.35 (d, J = 6.1 Hz, 5H), 7.32 - 7.23 (m, 5H), 4.79 - 4.70 (m, 1H), 4.66 - 4.59 (m, 1H), 4.55 (d, J = 4.7 Hz, 2H), 4.52 (d, J = 3.1 Hz, 1H), 4.29 (s, 1H), 3.85 (d, J = 6.7 Hz, 1H), 3.80 (d, J = 9.5 Hz, 1H), 3.73 (d, J = 6.5 Hz, 1H), 3.65 (d, J = 9.5 Hz, 1H), 2.44 (d, J = 9.1 Hz, 2H).

### Example 5: Synthesis of Compound I-1-5

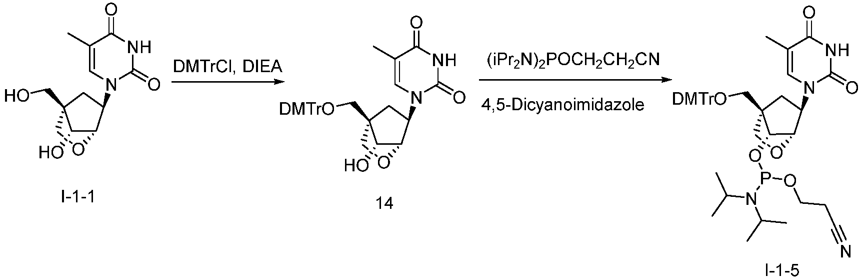

### (1) Synthesis of Compound 14

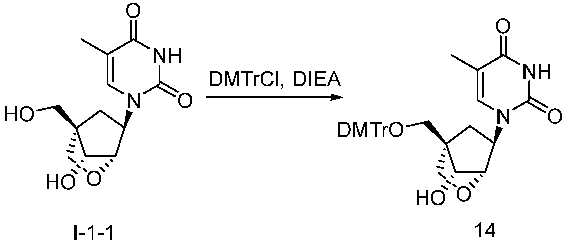

Under a nitrogen atmosphere, Compound I-1-1 (1.6 g, 6.0 mmol) and 4,4'-dimethoxytrityl chloride (4.65 g, 13.8 mmol) were dissolved in 30 ml of dry dichloromethane to obtain a mixture, a temperature was lowered to 0°C, and N,N-diisopropylethylamine (2.3 g, 18 mmol) was added dropwise, and stirred at room temperature for 2 h after the addition was completed. The reaction was monitored by the LCMS to ensure the reaction was completed, organic phases were washed with water and saturated brine in turn, and dried with anhydrous sodium sulfate, filtering and concentration were performed to obtain a crude product, the crude product was purified by the column chromatography with an eluent of dichloromethane: methanol = 40:1, and 2.8 g of a light yellow solid was obtained, with a yield of 86%. [M+1]⁺=571.2; 1H NMR (400 MHz, DMSO-d6) δ 11.26 (s, 1H), 7.42 (d, J = 1.4 Hz, 1H), 7.38 - 7.28 (m, 4H), 7.22 (ddt, J = 6.9, 4.8, 2.2 Hz, 5H), 6.92 - 6.86 (m, 4H), 5.10 (d, J = 4.6 Hz, 1H), 4.30 (dd, J = 9.9, 5.8 Hz, 1H), 3.96 (d, J = 4.7 Hz, 1H), 3.91 (s, 1H), 3.74 (s, 6H), 3.67 (s, 2H), 3.29 (d, J = 9.0 Hz, 1H), 3.12 (d, J = 9.0 Hz, 1H), 2.30 (dd, J = 13.5, 9.8 Hz, 1H), 1.78 (d, J = 1.1 Hz, 4H).

### (2) Synthesis of Compound I-1-5

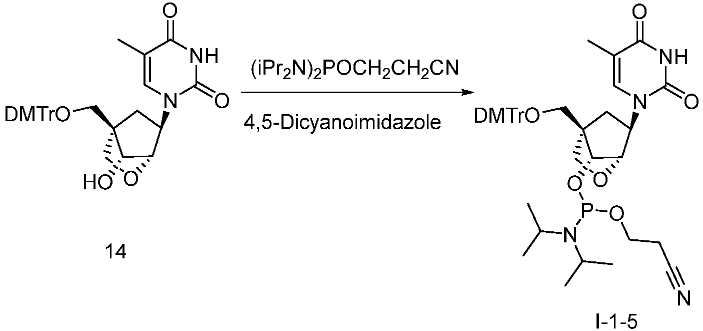

Under a nitrogen atmosphere, Compound 14 (700 mg, 1.2 mmol) and 4,5-dicyanoimidazole (354 mg, 3 mmol) were dissolved in 10 ml of dry dichloromethane, and bis(diisopropylamino)(2-cyanoethoxy)phosphine (904 mg, 3 mmol) was added dropwise at room temperature, and stirred continuously for 2 h. The reaction was monitored by the LCMS to ensure the reaction was completed, dichloromethane was added, organic phases were washed with saturated brine in turn, and dried with anhydrous sodium sulfate, filtering and concentration were performed to obtain a crude product, the crude product was separated by a high-pressure preparative liquid chromatography, C18 was used as a chromatographic column, a mobile phase was acetonitrile (containing 0.0035% diisopropylamine): water (containing 0.0035% diisopropylamine) = 8:2, and 560 mg of a white solid was obtained, with a yield of 60%. [M+1]⁺=771.3; ³¹PNMR: 148.30,148.04.

### Example 6: Synthesis of Compound I-1-6

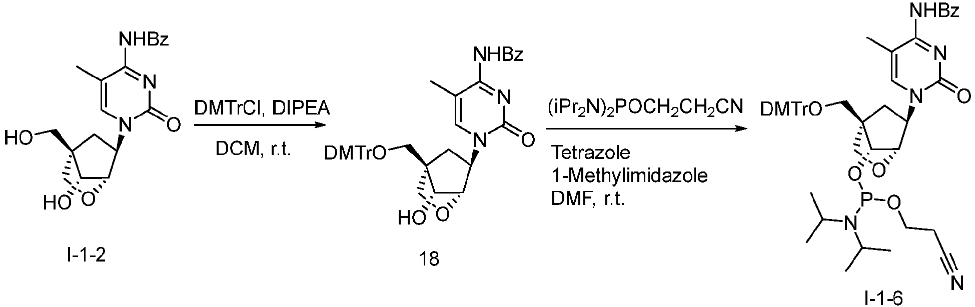

### (1) Synthesis of Compound 18

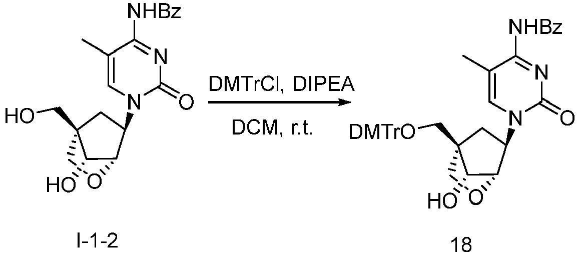

Under a nitrogen atmosphere, Compound I-1-2 (1.1 g, 3.12 mmol) and 4,4'-dimethoxytrityl chloride (2.3 g, 6.88 mmol) were dissolved in 20 ml of dry dichloromethane to obtain a mixture, a temperature was lowered to 0°C, and N,N-diisopropylethylamine (2.0 g, 15.6 mmol) was added dropwise, and stirred overnight at room temperature after the addition was completed. The reaction was monitored by the LCMS to ensure the reaction was completed, organic phases were washed with saturated sodium bicarbonate solution and saturated brine, and dried with saturated brine twice, filtering and concentration were performed to obtain a crude product, the crude product was purified by the column chromatography with an eluent of petroleum ether: ethyl acetate : TEA=3:1:0.1%, and 1.4 g of a white solid was obtained, with a yield of 70%. [M+1]⁺=674.3; 1H NMR (400 MHz, DMSO-d6) δ 13.18 (s, 1H), 8.20 (s, 2H), 7.79 (s, 1H), 7.59 (t, J = 7.3 Hz, 1H), 7.50 (t, J = 7.5 Hz, 2H), 7.40 - 7.30 (m, 5H), 7.24 (dd, J = 8.9, 3.2 Hz, 4H), 6.93 - 6.89 (m, 4H), 5.15 (d, J = 4.7 Hz, 1H), 4.38 (dd, J = 9.7, 5.8 Hz, 1H), 4.04 (d, J = 7.1 Hz, 1H), 4.02 - 3.98 (m, 1H), 3.75 (s, 8H), 3.29 (s, 1H), 3.16 (d, J = 9.0 Hz, 1H), 2.40 (t, J = 11.7 Hz, 1H), 1.99 (s, 3H), 1.92 - 1.86 (m, 1H).

### (2) Synthesis of Compound I-1-6

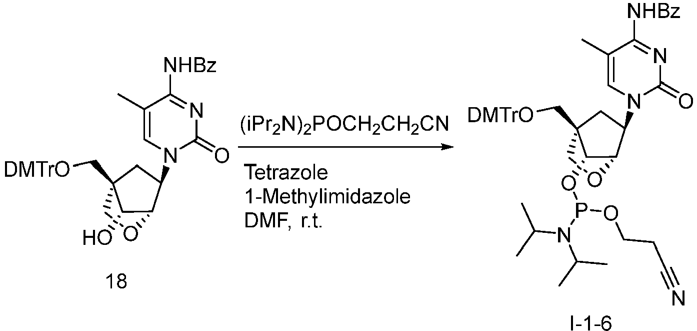

Under a nitrogen atmosphere, Compound 18 (500 mg, 0.74 mmol), tetrazole (154 mg, 2.2 mmol) and N-methylimidazole (61 mg, 0.74 mmol) were dissolved in 5 ml of dry N,N-dimethylformamide, and bis(diisopropylamino)(2-cyanoethoxy)phosphine (446 mg, 1.48 mmol) was added dropwise at room temperature, and stirred continuously for 2 h. The reaction was monitored by the LCMS to ensure the reaction was completed, separation was performed with a high-pressure preparative liquid chromatography, C18 was used as a chromatographic column, and a mobile phase was 100% acetonitrile to obtain 320 mg of a white solid, with a yield of 49%. [M+1]⁺=874.4; ³¹PNMR: 148.4, 148.2.

### Example 7: Synthesis of Compound I-1-7

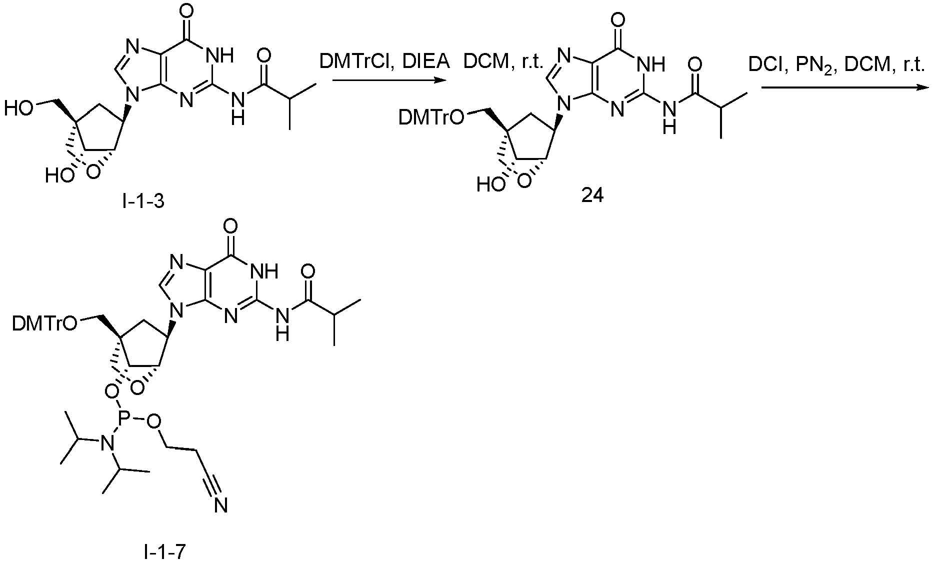

### (1) Synthesis of Compound 24

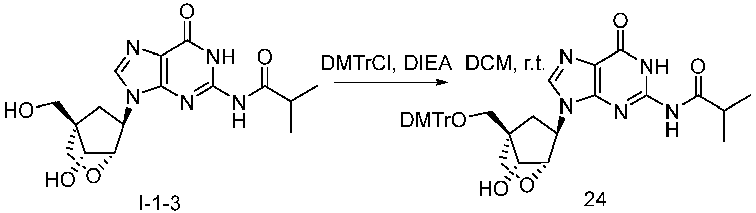

Under a nitrogen atmosphere, Compound I-1-3 (3.1 g, 9.1 mmol) and 4,4'-dimethoxytrityl chloride (6.8 g, 20.2 mmol) were dissolved in 30 ml of dry dichloromethane to obtain a mixture, a temperature was lowered to 0°C, and N,N-diisopropylethylamine (5.89 g, 45.5 mmol) was added dropwise, heated up to room temperature and stirred for 2 h after the addition was completed. The reaction was monitored by the LCMS to ensure the reaction was completed, concentration was performed to obtain a crude product, the crude product was purified by the column chromatography with an eluent of dichloromethane: methanol : triethylamine =30:1:0.1%, and 4.6 g of a light yellow solid was obtained, with a yield of 76%. [M+1]⁺=666.3; 1H NMR (400 MHz, Chloroform-d) δ 12.10 (s, 1H), 9.40 (s, 1H), 7.75 (s, 1H), 7.46 - 7.40 (m, 2H), 7.34 - 7.25 (m, 5H), 7.23 - 7.17 (m, 2H), 6.83 (ddd, J = 9.0, 4.2, 2.0 Hz, 4H), 4.66 (s, 1H), 4.48 (t, J = 7.6 Hz, 1H), 4.27 (s, 1H), 3.95 (d, J = 7.1 Hz, 1H), 3.82 (s, 1H), 3.78 (s, 6H), 3.68 (d, J = 7.0 Hz, 1H), 3.46 (d, J = 9.6 Hz, 1H), 3.27 (d, J = 9.6 Hz, 1H), 2.53 (p, J = 6.9 Hz, 1H), 2.32 (d, J = 7.6 Hz, 2H), 1.19 (dd, J = 9.7, 6.8 Hz, 6H).

### (2) Synthesis of Compound I-1-7

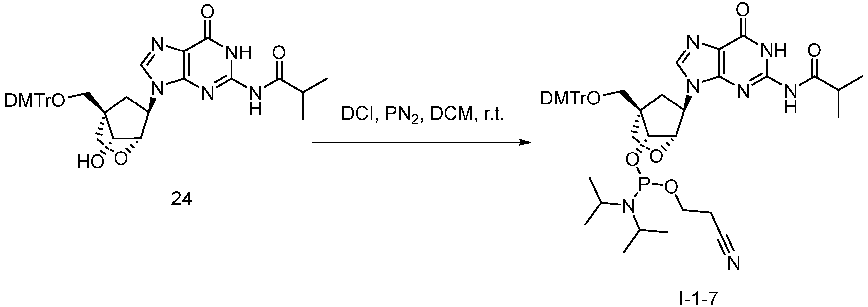

Under a nitrogen atmosphere, Compound 24 (1.0 g, 1.5 mmol) and 4,5-dicyanoimidazole (443 mg, 3.75 mmol) were dissolved in 10 ml of dry dichloromethane, and bis(diisopropylamino)(2-cyanoethoxy)phosphine (1.1 g, 3.75 mmol) was added dropwise at room temperature, and stirred continuously for 2 h. The reaction was monitored by the LCMS to ensure the reaction was completed, dichloromethane and 0.1 ml of triethylamine were added, organic phases were washed with saturated brine in turn, and dried with anhydrous sodium sulfate, filtering and concentration were performed to obtain a crude product, the crude product was separated by a high-pressure preparative liquid chromatography, C18 was used as a chromatographic column, a mobile phase was acetonitrile (containing 0.0035% diisopropylamine): water (containing 0.0035% diisopropylamine) = 7:3, and 660 mg of a white solid was obtained, with a yield of 51%. [M+1]⁺=866.4; ³¹PNMR: 147.27, 147.17.

### Example 8: Synthesis of Compound I-1-8

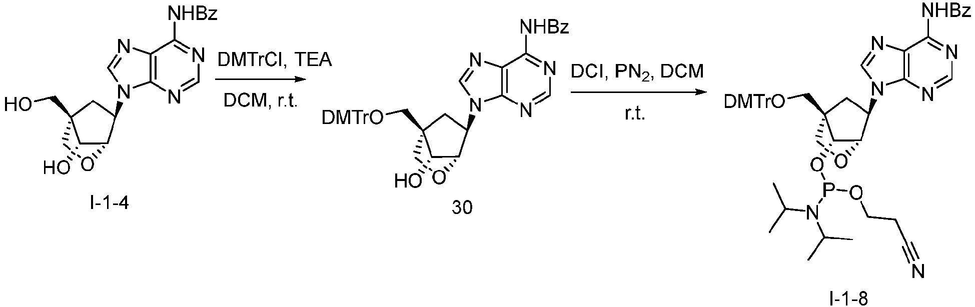

### (1) Synthesis of Compound 30

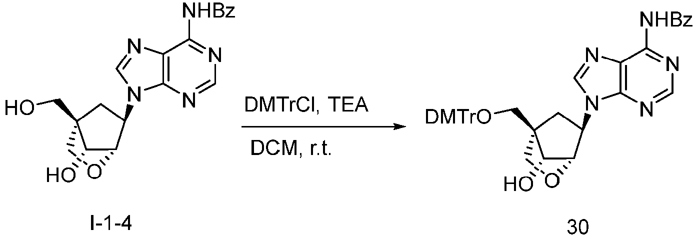

Under a nitrogen atmosphere, Compound I-1-4 (1 g, 2.6 mmol) and 4,4'-dimethoxytrityl chloride (1.9 g, 5.72 mmol) were dissolved in dry dichloromethane to obtain a mixture, a temperature was lowered to 0°C, and triethylamine (1.3 g, 13 mmol) was added dropwise, heated up to room temperature and stirred for 2 h after the addition was completed. The reaction was monitored by the LCMS to ensure the reaction was completed, concentration were performed under a reduced pressure to obtain a crude product, the crude product was purified by the column chromatography with an eluent of dichloromethane : ethyl acetate : triethylamine =1:1:0.1%, and 1.3 g of a light yellow solid was obtained, with a yield of 72.2%. [M+1]⁺=684.3; 1H NMR (400 MHz, DMSO-d6) δ 11.16 (s, 1H), 8.76 (s, 1H), 8.62 (s, 1H), 8.05 (d, J = 7.7 Hz, 2H), 7.65 (t, J = 7.4 Hz, 1H), 7.56 (t, J = 7.6 Hz, 2H), 7.41 (d, J = 7.8 Hz, 2H), 7.33 (t, J = 7.6 Hz, 2H), 7.30 - 7.21 (m, 5H), 6.96 - 6.87 (m, 4H), 5.20 (d, J = 4.6 Hz, 1H), 4.75 (dd, J = 9.8, 5.5 Hz, 1H), 4.36 (d, J = 4.7 Hz, 1H), 4.11 (s, 1H), 3.81 (d, J = 6.5 Hz, 1H), 3.75 (s, 6H), 3.40 (d, J = 9.1 Hz, 1H), 3.21 - 3.16 (m, 2H), 2.57 - 2.52 (m, 1H), 2.46-2.39(m, 1H).\

### (2) Synthesis of Compound I-1-8

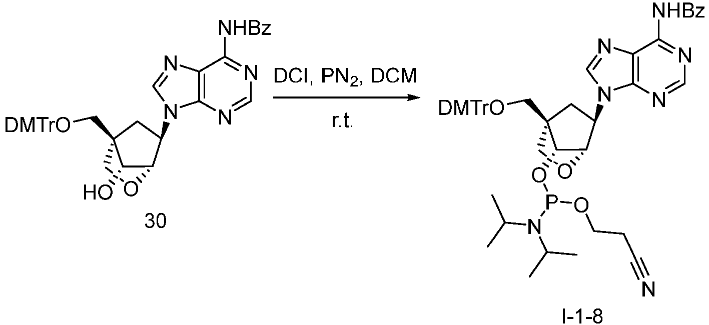

Under a nitrogen atmosphere, Compound 30 (800 mg, 1.17 mmol) and 4,5-dicyanoimidazole (345 mg, 2.92 mmol) were dissolved in 10 ml of dry dichloromethane, and bis(diisopropylamino)(2-cyanoethoxy)phosphine (880mg, 2.92 mmol) was added dropwise at room temperature, and stirred continuously for 2 h. The reaction was monitored by the LCMS to ensure the reaction was completed, dichloromethane and 0.1 ml of triethylamine were added, organic phases were washed with saturated brine in turn, and dried with anhydrous sodium sulfate, filtering and concentration were performed to obtain a crude product, the crude product was separated by a high-pressure preparative liquid chromatography, C18 was used as a chromatographic column, a mobile phase was acetonitrile (containing 0.0035% diisopropylamine): water (containing 0.0035% diisopropylamine) = 8:2, and 550 mg of a white solid was obtained, with a yield of 53%. [M+1]⁺=884.4; ³¹PNMR: 148.40, 148.31.

### Example 9: Synthesis of Compound I-1-9

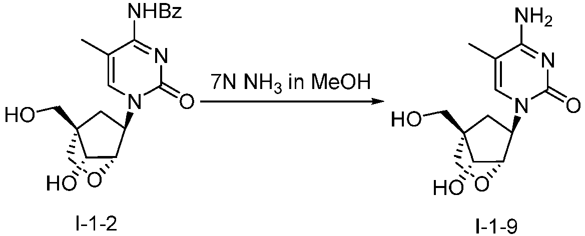

Under a nitrogen atmosphere, Compound I-1-2 (30 mg, 0.081 mmol) was dissolved in 3 ml of 7N ammonia methanol solution, and stirred at room temperature for 4 h. The reaction was monitored by the LCMS to ensure the reaction was completed, the mixture was concentrated, and washed with a small amount of methanol, and filtering was performed to obtain 18 mg of a white solid, with a yield of 83%. [M+1]⁺=268.1; 1H NMR (400 MHz, DMSO-d6) δ 7.34 (s, 1H), 6.90 (d, J = 171.7 Hz, 2H), 5.04 (d, J = 4.1 Hz, 1H), 4.53 (t, J = 5.3 Hz, 1H), 4.27 (dd, J = 9.7, 5.6 Hz, 1H), 3.97 - 3.78 (m, 2H), 3.72 - 3.56 (m, 2H), 3.54 - 3.40 (m, 2H), 2.02 (dd, J = 13.6, 9.7 Hz, 1H), 1.86 (s, 3H), 1.72 (ddd, J = 13.9, 5.8, 2.6 Hz, 1H).

### Example 10: Synthesis of Compound I-1-10

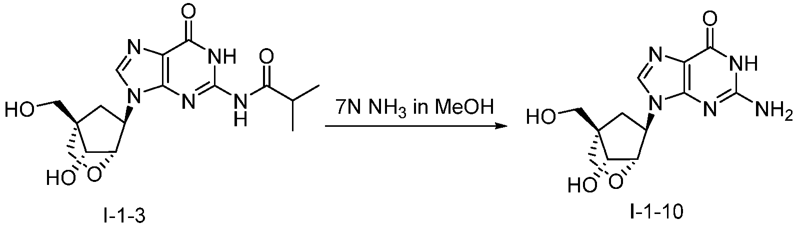

Under a nitrogen atmosphere, Compound I-1-3 (50 mg, 0.14 mmol) was dissolved in 5 ml of 7N ammonia methanol solution, and stirred at room temperature for 3 d. The reaction was monitored by the LCMS to ensure the reaction was completed, the mixture was concentrated, and washed with a small amount of methanol, and filtering was performed to obtain 35mg of a white solid, with a yield of 87%. [M+1]⁺=294.1; 1H NMR (400 MHz, DMSO-d6) δ 10.58 (s, 1H), 7.81 (s, 1H), 6.48 (s, 2H), 5.21 - 5.02 (m, 1H), 4.56 (s, 1H), 4.33 (dd, J = 9.6, 5.7 Hz, 1H), 4.02 (s, 1H), 3.90 (s, 1H), 3.79 - 3.63 (m, 2H), 3.53 (dd, J = 13.5, 8.5 Hz, 2H), 2.16 (qd, J = 13.7, 7.2 Hz, 2H).

### Example 11: Synthesis of Compound I-2-1

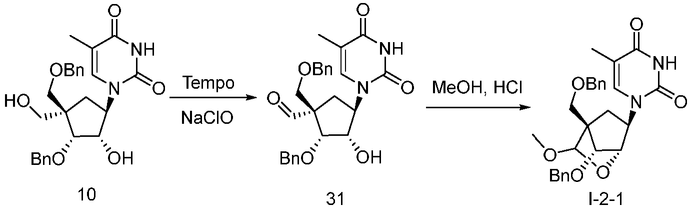

### (1) Synthesis of Compound 31

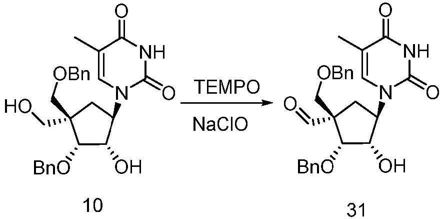

Under a nitrogen atmosphere, Compound 10 (1.6 g, 3.4 mmol) was dissolved in 30 ml of dichloromethane and 30 ml of water, 2,2,6,6-tetramethylpiperidinyl oxide (55 mg, 0.35 mmol), and potassium bromide (40 mg, 0.34 mmol) were added to obtain a mixture, a temperature was lowered to 0°C, 10% sodium hypochlorite solution (3.5 g, 4.8 mmol) was added dropwise (pH = 9 was adjusted with saturated sodium bicarbonate solution), and stirred continuously for 1 h after the addition was completed. The reaction was monitored by the LCMS to ensure the reaction was completed, sodium thiosulfate was added to quench, phase separation was performed, and the mixture was washed and dried, filtering and concentration were performed to obtain a crude product, the crude product was purified by the column chromatography with an eluent of ethyl acetate : petroleum ether =1:2 - 1:1, and 950 mg of a solid was obtained, with a yield of 60%. [M+1]⁺=465.2; 1H NMR (400 MHz, DMSO-d6) δ 11.22 (d, J = 2.2 Hz, 1H), 9.57 (d, J = 2.1 Hz, 1H), 7.85 - 7.40 (m, 1H), 7.39 - 7.14 (m, 10H), 5.37 (dd, J = 5.8, 2.1 Hz, 1H), 4.86 (dd, J = 11.8, 2.0 Hz, 1H), 4.65 (d, J = 9.8 Hz, 1H), 4.58 - 4.54 (m, 1H), 4.54 - 4.45 (m, 2H), 4.40 (dt, J = 5.3, 2.4 Hz, 1H), 4.12 (dd, J = 4.7, 2.0 Hz, 1H), 3.88 (dd, J = 9.2, 2.1 Hz, 1H), 3.61 (dd, J = 9.2, 2.1 Hz, 1H), 1.83 - 1.71 (m, 3H), 1.55 - 1.42 (m, 1H), 1.37 - 1.11 (m, 1H).

### (2) Synthesis of Compound I-2-1

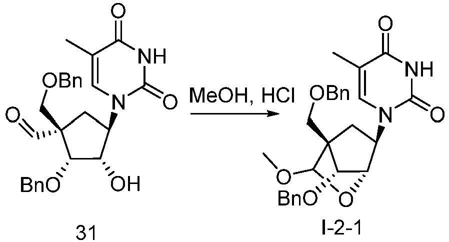

Under a nitrogen atmosphere, Compound 31 (400 mg, 0.86 mmol) was dissolved in 5 ml of methanol, 20 µl of 30% hydrogen chloride methanol solution was added dropwise to obtain a mixture, and the mixture was stirred at room temperature for 4 h. Concentration were performed to obtain a crude product, the crude product was purified by the column chromatography with an eluent of petroleum ether: ethyl acetate = 1:5, and 200 mg of substance was obtained, with a yield of 49%. [M+1]⁺=479.2; 1H NMR (400 MHz, DMSO-d6) δ 11.28 (s, 1H), 7.37 - 7.27 (m, 10H), 7.16 (d, J = 1.4 Hz, 1H), 4.97 (s, 1H), 4.69 - 4.57 (m, 2H), 4.53 (d, J = 2.5 Hz, 2H), 4.41 (s, 1H), 4.36 (dd, J = 9.6, 6.0 Hz, 1H), 4.06 (s, 1H), 3.69 (d, J = 9.8 Hz, 1H), 3.46 (d, J = 9.8 Hz, 1H), 3.39 (s, 3H), 2.38 (dd, J = 13.4, 9.6 Hz, 1H), 1.75 (d, J = 1.1 Hz, 3H), 1.66 (dd, J = 13.3, 6.1 Hz, 1H).

### Example 12: Synthesis of Compound I-2-1

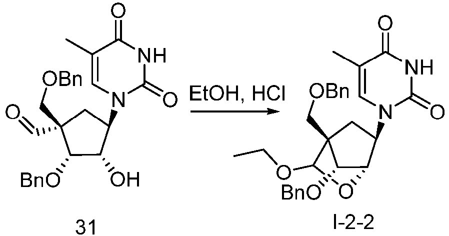

Under a nitrogen atmosphere, Compound 31 (400 mg, 0.86 mmol) was dissolved in 5 ml of ethanol absolute, 20 µl of 30% hydrogen chloride ethanol solution was added dropwise to obtain a mixture, and the mixture was stirred at room temperature for 4 h. Concentration were performed to obtain a crude product, the crude product was purified by the column chromatography with an eluent of petroleum ether: ethyl acetate = 1:5, and 210mg of substance was obtained, with a yield of 50%. [M+1]⁺=493.2; 1H NMR (400 MHz, DMSO-d6) δ 11.27 (s, 1H), 7.38 - 7.26 (m, 10H), 7.15 (d, J = 1.4 Hz, 1H), 5.08 (s, 1H), 4.68 - 4.57 (m, 2H), 4.57 - 4.49 (m, 2H), 4.40 - 4.33 (m, 2H), 4.03 (s, 1H), 3.78 - 3.67 (m, 2H), 3.58 - 3.48 (m, 1H), 3.46 (d, J = 9.8 Hz, 1H), 2.41 (dd, J = 13.4, 9.6 Hz, 1H), 1.74 (d, J = 1.1 Hz, 3H), 1.66 (dd, J = 13.4, 6.0 Hz, 1H), 1.14 (t, J = 7.0 Hz, 3H).

### Example 13: Synthesis of Compound I-4-1

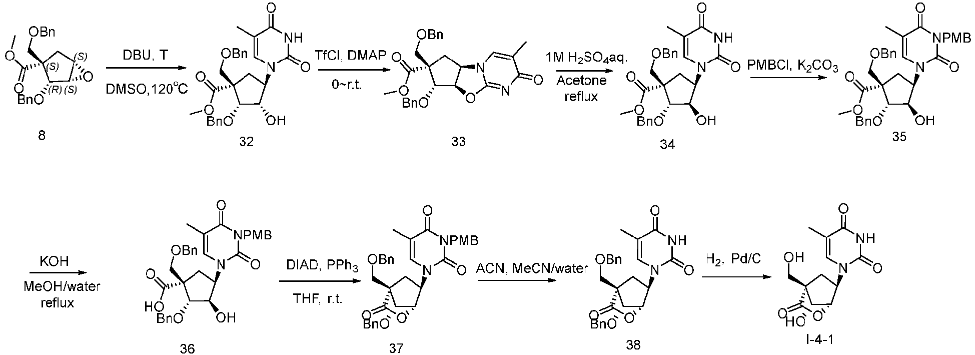

### (1) Synthesis of Compound 32

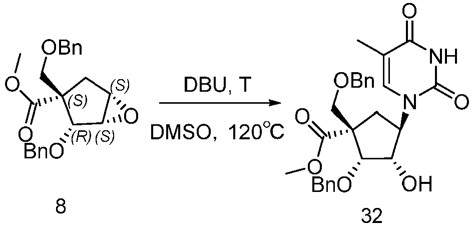

Under a nitrogen atmosphere, Compound 8 (10 g, 27.1 mmol) and potassium carbonate (13.5 g, 108.4 mmol) were suspended in 100 ml of dry dimethyl sulfoxide, 1,8-diazabicyclo[5.4.0]undec-7-ene (41 g, 271 mmol) was added and heated to 120 °C and reacted for 3 h to obtain a mixture. The reaction was monitored by the LCMS to ensure the reaction was halfway, a temperature was lowered, water was added, extraction was performed twice with ethyl acetate, organic phases were combined and washed with saturated brine in turn, and dried with anhydrous sodium sulfate, filtering and concentration were performed to obtain a crude product, the crude product was purified by the column chromatography with an eluent of dichloromethane: methanol =50:1, 4.2 g of substance was obtained, with a yield of 31%, and 5.4 g of Compound 8 was recovered. [M+1]⁺=495.2; 1H NMR (400 MHz, DMSO-d6) δ 11.21 (s, 1H), 7.48 (s, 1H), 7.40 - 7.24 (m, 10H), 5.35 (d, J = 6.0 Hz, 1H), 4.86 (d, J = 11.4 Hz, 1H), 4.74 (q, J = 10.0 Hz, 1H), 4.56 (d, J = 12.1 Hz, 1H), 4.51 - 4.40 (m, 4H), 3.86 (d, J = 8.5 Hz, 1H), 3.78 (d, J = 4.2 Hz, 1H), 3.70 (d, J = 8.5 Hz, 1H), 3.54 (s, 3H), 2.70 (dd, J = 13.7, 9.7 Hz, 1H), 1.80 - 1.68 (m, 4H).

### (1) Synthesis of Compound 33

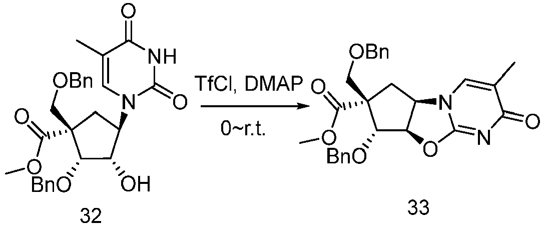

Under a nitrogen atmosphere, Compound 32 (4.2 g, 8.5 mmol) and 4-dimethylaminopyridine (4.1 g, 33.6 mmol) were dissolved in 50 ml of dichloromethane to obtain a mixture, a temperature was lowered to 0°C, trifluoromethylsulfonyl chloride (4.3 g, 25.5 mmol) was added dropwise, and a temperature was naturally raised to room temperature and stirred for 2 h after the addition was completed. The reaction was monitored by the LCMS to ensure the reaction was completed, water was added, phase separation was performed, and dried with anhydrous sodium sulfate to obtain a crude product, the crude product was directly subjected to the next step of reaction without purification. [M+1]⁺=477.2.

### (2) Synthesis of Compound 34

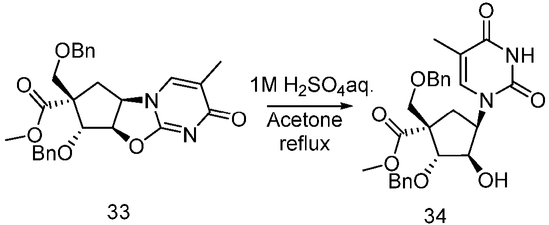

Under a nitrogen atmosphere, 100 ml of acetone and 100 ml of 1 M sulfuric acid aqueous solution were added to the crude product of Compound 33 obtained in the previous step, and heated under reflux to react overnight. The reaction was monitored by the LCMS to ensure the reaction was completed, acetone was concentrated under a reduced pressure, residue was extracted twice with ethyl acetate, organic phases were combined and washed with saturated brine in turn, and dried with anhydrous sodium sulfate, filtering and concentration were performed to obtain a crude product, the crude product was purified by the column chromatography with an eluent of petroleum ether: ethyl acetate = 2:1, and 3.4 g of substance was obtained, with a yield of 80% over two steps. [M+1]⁺=495.2; 1H NMR (400 MHz, Chloroform-d) δ 8.34 (s, 1H), 7.42 - 7.23 (m, 10H), 5.18 (ddd, J = 10.6, 8.9, 5.6 Hz, 1H), 4.69 (d, J = 11.9 Hz, 1H), 4.58 (s, 2H), 4.56 (d, J = 11.9 Hz, 1H), 4.39 (dd, J = 5.8, 3.3 Hz, 1H), 3.98 - 3.87 (m, 2H), 3.77 - 3.72 (m, 1H), 3.69 (s, 3H), 2.67 (dd, J = 13.6, 8.9 Hz, 1H), 2.22 (dd, J = 13.6, 10.7 Hz, 1H), 1.81 (s, 3H).

### (3) Synthesis of Compound 35

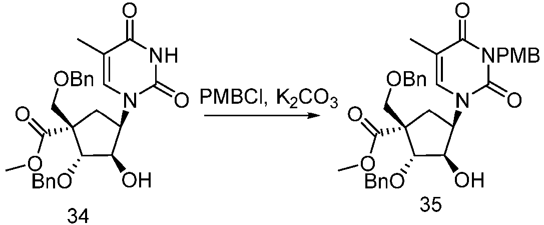

Under a nitrogen atmosphere, Compound 34 (3.4 g, 6.9 mmol), potassium carbonate (2.8 g, 20.6 mmol), and 4-methoxybenzyl chloride (1.6 g, 10.4 mmol) were dissolved in 25 ml of dry N,N-dimethylformamide to obtain a mixture, the mixture was heated to 50°C and stirred for reaction for 2 h. The reaction was monitored by the LCMS to ensure the reaction was completed, a temperature was lowered, water was added to dilute, extraction was performed twice with ethyl acetate, organic phases were combined and washed with saturated brine in turn, and dried with anhydrous sodium sulfate, filtering and concentration were performed to obtain a crude product, the crude product was purified by the column chromatography with an eluent of petroleum ether: ethyl acetate = 1:3, and 3.9 g of substance was obtained, with a yield of 93%. [M+1]⁺=615.3.

### (4) Synthesis of Compound 36

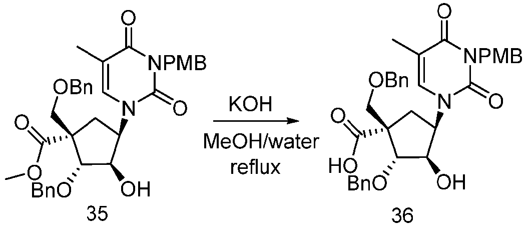

Under a nitrogen atmosphere, Compound 35 (500 mg, 0.3 mmol) and potassium hydroxide (138 mg, 0.9 mmol) were dissolved in a mixed solvent of 5 ml of methanol and 1 ml of purified water and heated under reflux overnight. The reaction was monitored by the LCMS to ensure the reaction was completed, a temperature was lowered, reaction solution was poured into water, a pH was adjusted to 3-4 with hydrochloric acid, extraction was performed twice with ethyl acetate, organic phases were combined, and dried with anhydrous sodium sulfate, filtering and concentration were performed to obtain a crude product, the crude product was purified by the column chromatography with an eluent of petroleum ether: ethyl acetate = 1:1, and 280 mg of substance was obtained, with a yield of 65%. [M+1]⁺=601.2.

### (5) Synthesis of Compound 37

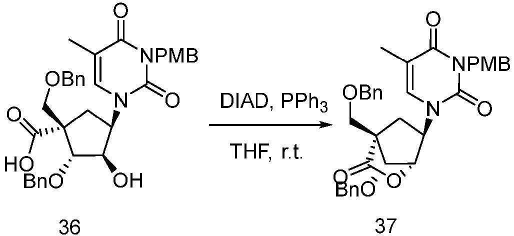

Under a nitrogen atmosphere, Compound 36 (280 mg, 0.2 mmol), triphenylphosphine (366 mg, 0.6 mmol) and diisopropyl azodicarboxylate (280 mg, 0.6 mmol) were dissolved in 5 ml of dry tetrahydrofuran and stirred at room temperature overnight. The reaction was monitored by the LCMS to ensure the reaction was completed, reaction solution was concentrated to obtain a crude product, the crude product was purified by the column chromatography with an eluent of petroleum ether: ethyl acetate =1:4, and 240 mg of a substance was obtained, with a yield of 90%. [M+1]⁺=583.2.

### (6) Synthesis of Compound 38

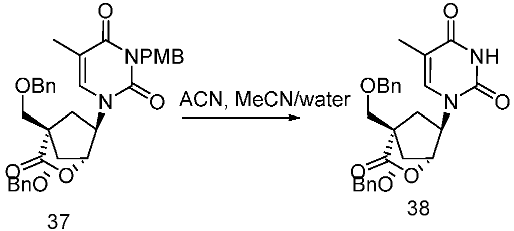

Under a nitrogen atmosphere, Compound 37 (240 mg, 0.16 mmol) and ceric ammonium nitrate (670 mg, 0.48 mmol) were dissolved in 3 ml of acetonitrile and 1 ml of purified water, heated to 60 °C and stirred for 6 h. The reaction was monitored by the LCMS to ensure the reaction was completed, a temperature was lowered, water was added to the reaction solution, extraction was performed twice with ethyl acetate, organic phases were combined and washed with saturated brine in turn, and dried with anhydrous sodium sulfate, filtering and concentration were performed to obtain a crude product, the crude product was purified by the column chromatography with an eluent of petroleum ether: ethyl acetate = 1:1, and 65 mg of substance was obtained, with a yield of 33%. [M+1]⁺=463.2.

### (7) Synthesis of Compound I-4-1

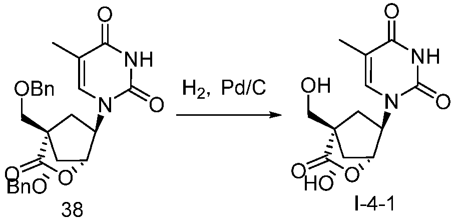

Compound 38 (65 mg, 0.05 mmol) and 65 mg of 10% palladium carbon (containing 55% water) were suspended in 5 ml of methanol and 0.5 ml of glacial acetic acid to obtain a mixture, hydrogen was purged three times, and the mixture was stirred at room temperature for 4 days. The reaction of raw material was monitored by the LCMS to ensure that the reaction was basically completed, filtering was performed to obtain a filter cake, the filter cake was washed with 50% methanol aqueous solution and concentrated to obtain a crude product, the crude product was purified by the column chromatography with dichloromethane : methanol = 20:1, and 8 mg of a white solid was obtained, with a yield of 20%. [M+1]⁺=283.1. 1H NMR (400 MHz, DMSO-d6) δ 11.39 (s, 1H), 7.45 (s, 1H), 5.84 (d, J = 3.4 Hz, 1H), 4.83 (s, 1H), 4.78 (t, J = 5.5 Hz, 1H), 4.41 - 4.36 (m, 1H), 4.36 - 4.33 (m, 1H), 3.70 (dd, J = 11.6, 5.3 Hz, 1H), 3.62 (dd, J = 11.6, 5.5 Hz, 1H), 2.22 (dd, J = 14.0, 5.5 Hz, 1H), 2.03 (dd, J = 14.0, 9.5 Hz, 1H), 1.81 (s, 3H).

### Example 14: Synthesis of Compound I-3-1

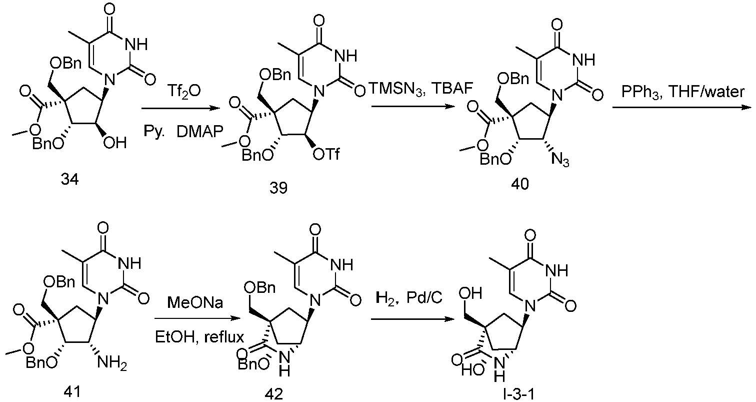

### (1) Synthesis of Compound 39

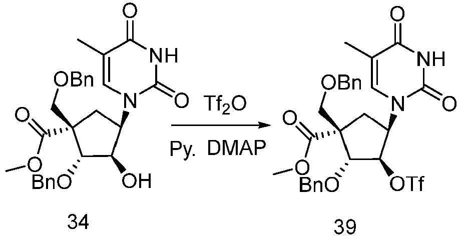

Under a nitrogen atmosphere, Compound 34 (2.11 g, 4.27 mmol) and 4-dimethylaminopyridine (2.08 g, 17.07 mmol) were dissolved in 24 ml of dry dichloromethane and 6 ml of pyridine to obtain a mixture, the mixture was cooled to 0°C, and trifluoromethanesulfonic anhydride (2.41 g, 8.54 mmol) was added dropwise, and continuously stirred for 3 h after the addition was completed. The reaction was monitored by the LCMS to ensure the reaction was completed, organic phases were washed with 10% citric acid aqueous solution and saturated brine in turn, filtering and concentration were performed to obtain a crude product, the crude product was purified by the column chromatography with an eluent of ethyl acetate : petroleum ether =1:3 - 1:1, and 1.62 g of a solid was obtained, with a yield of 61%. [M+1]⁺=627.2.

### (2) Synthesis of Compound 40

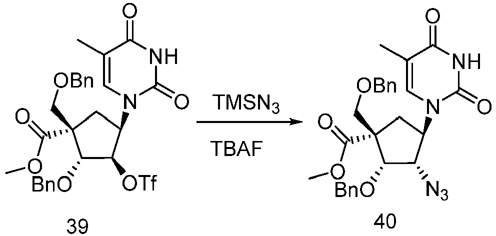

Under a nitrogen atmosphere, Compound 39 (870 mg, 1.39 mmol) and azidotrimethylsilane (480 mg, 4.17 mmol) were dissolved in 8 ml of dry tetrahydrofuran, and 4.2 ml of tetrahydrofuran solution of 1M tetrabutylammonium fluoride was added dropwise to obtain a mixture, and the mixture was stirred at room temperature overnight. The reaction was monitored by the LCMS to ensure the reaction was completed, concentration were performed under a reduced pressure to obtain a crude product, the crude product was purified by the column chromatography with an eluent of ethyl acetate : petroleum ether =1:3 - 1:1, and 630 mg of a substance was obtained. [M+1]⁺=520.2. 1H NMR (400 MHz, Chloroform-d) δ 8.66 (s, 1H), 7.46 - 7.30 (m, 10H), 7.03 (s, 1H), 5.02 (q, J = 10.1 Hz, 1H), 4.83 (d, J = 10.8 Hz, 1H), 4.64 (d, J = 11.7 Hz, 1H), 4.59 (d, J = 10.9 Hz, 1H), 4.53 (d, J = 11.8 Hz, 1H), 4.20 (dd, J = 10.7, 4.6 Hz, 1H), 4.09 (d, J = 4.7 Hz, 1H), 3.85 (d, J = 8.7 Hz, 1H), 3.71 (d, J = 8.7 Hz, 1H), 3.62 (s, 3H), 3.02 (dd, J = 14.0, 10.0 Hz, 1H), 2.11 (dd, J = 13.9, 9.7 Hz, 1H), 1.75 (s, 3H).

### (3) Synthesis of Compound 41

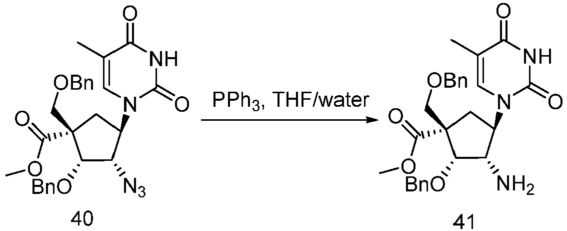

Under a nitrogen atmosphere, Compound 40 (1.0 g, 1.92 mmol) and triphenylphosphine (0.6 g, 2.3 mmol) were dissolved in 6 ml of tetrahydrofuran and 2 ml of purified water to obtain a mixture, and the mixture was stirred at room temperature for 2 h and then continuously stirred at 50 °C for 6 h. The reaction was monitored by the LCMS to ensure the reaction was completed, concentration was performed to obtain a crude product, the crude product was purified by the column chromatography with an eluent of dichloromethane: methanol =50:1- 10:1, and 600 mg of a substance was obtained. [M+1]⁺=494.2.

### (4) Synthesis of Compound 42

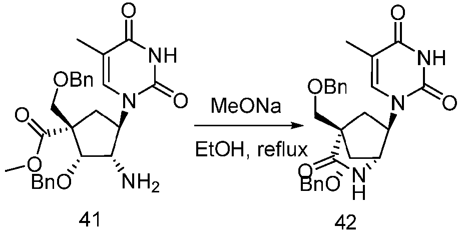

Under a nitrogen atmosphere, Compound 41 (180 mg, 0.35 mmol) and sodium methoxide (95 mg, 1.7 mmol) were dissolved in 10 ml of anhydrous ethanol for reflux and stirred for 10 h. The reaction was monitored by the LCMS to ensure the reaction was completed, a temperature was lowered to room temperature, hydrochloric acid was used to adjust a pH < 7, concentration was performed to obtain a crude product, the crude product was purified by the column chromatography with an eluent of dichloromethane: methanol :=100:1-20:1, and 80 mg of substance was obtained. [M+1]⁺=462.2; 1H NMR (400 MHz, DMSO-d6) δ 11.33 (s, 1H), 8.13 (s, 1H), 7.43 - 7.26 (m, 10H), 7.22 (s, 1H), 4.69 - 4.48 (m, 4H), 4.21 (s, 1H), 4.18 (dd, J = 8.9, 4.5 Hz, 1H), 3.97 (s, 1H), 3.64 (d, J = 10.2 Hz, 1H), 3.57 (d, J = 10.2 Hz, 1H), 2.05 - 1.99 (m, 1H), 1.96 - 1.88 (m, 1H), 1.75 (s, 3H).

### (5) Synthesis of Compound I-3-1

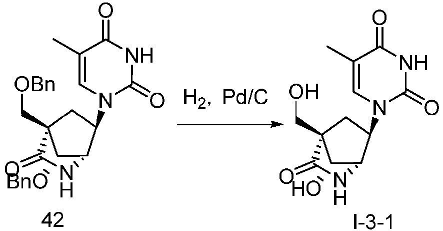

Compound 42 (25 mg, 0.054 mmol) and 25 mg of 10% palladium carbon (containing 55% water) were suspended in 5 ml of methanol and 1 ml of acetic acid to obtain a mixture, hydrogen was purged three times, and the mixture was stirred at room temperature overnight. The reaction was monitored by the LCMS to ensure that the reaction was basically completed, filtering was performed to obtain a filter cake, the filter cake was washed with 50% methanol aqueous solution and concentrated to obtain a crude product, the crude product was purified by the column chromatography with dichloromethane : methanol = 5:1, and 8 mg of a white solid was obtained, with a yield of 60%. [M+1]⁺=282.1; 1H NMR (400 MHz, DMSO-d6) δ 11.33 (s, 1H), 7.96 (s, 1H), 7.50 (d, J = 1.4 Hz, 1H), 5.35 (d, J = 3.8 Hz, 1H), 4.51 (t, J = 5.5 Hz, 1H), 4.13(dd, J=9.2, 4.3Hz, 1H), 4.07 (dd, J = 3.5, 1.8 Hz, 1H), 3.79 (d, J = 2.1 Hz, 1H), 3.63 (dd, J = 11.6, 5.5 Hz, 1H), 3.55 (dd, J = 11.6, 5.6 Hz, 1H), 1.96 (dd, J = 14.0, 4.4 Hz, 1H), 1.82 (s, 3H), 1.81 - 1.75 (m, 1H).

### Example 15: Synthesis and Purification of Oigonucleotide Analogs

By using an automated oligonucleotide synthesizer, multi-step solid phase synthesis, such as deprotection, coupling, capping, oxidation, and ammonolysis, of an oligonucleotide analog containing 10 mer derived from Compound I-1-5 was performed at a scale of 0.2 µmol through a solid phase carrier and a phosphoramidite nucleoside monomer, as shown in Table 1, where a sequence was represented by "X".

A crude oligonucleotide analog was purified using ion-pair reversed-phase liquid chromatography, a chromatographic column was a NanoQ-15L ion exchange column (9.5 mL, 8×190 mm), with a flow rate of 2 mL/min, a mobile phase A was a 10 mM sodium hydroxide solution, a mobile phase B was a solution composed of 10 mM sodium hydroxide and 2.0 M sodium chloride, and a gradient elution was performed to obtain oligonucleotide with purity that meets the specified requirements.

**Table 1**

| Oligonucleotide | | Purity (%) | LC-MS | |
|---|---|---|---|---|
| | | | Calculated value (M-H⁻) | Measured value (M-H⁻) |
| Antisense strand 1 | 5'-TTTTTTTTTX-3' | 97.9 | 3005.02 | 3005.8 |
| Antisense strand 2 | 5'-TTTTTTTXTT-3' | 94.5 | 3005.02 | 3005.2 |
| Control antisense strand | 5'-TTTTTTTTTT-3' | 98.4 | 2978.98 | 2979.2 |

### Example 16 Melting Temperature (Tm) Test

After annealing the antisense strand 1, the antisense strand 2, the control antisense strand, and the sense strand (5'-AAAAAAAAAA-3') synthesized in Example 15, the hybridization ability of the antisense strands was assessed by measuring Tm values.

A mixture of 1 mL of the sense strand (0.5 OD/mL) and 1 mL of the antisense strand (0.5 OD/mL) was mixed and stirred evenly, heated at 90°C for 10 min, and then cooled to room temperature. A Tm value of the sample was measured using an ultraviolet spectrophotometer (Agilent, Cary 3500 UV-Vis), the sample was placed in a cuvette with a cap and a temperature probe, a temperature was set to be raised from 20-25°C to 70-80°C at a rate of 0.5°C or 1.0°C per minute, and ultraviolet absorption at 260 nm was measured at an interval of 0.5°C.

**Table 2**

| Antisense strand | Tm (°C) | ΔTm/mod.(°C) |
|---|---|---|
| Antisense strand 1 | 46.3 | +3.3 |
| Antisense strand 2 | 45.1 | +2.1 |
| Control antisense strand | 43 | - |

Results in Table 2 show that a natural oligonucleotide containing the carbocyclic nucleoside Compound X in the present disclosure had a higher Tm value relative to a single-stranded RNA oligonucleotide compared with the natural oligonucleotide, demonstrating high binding affinity.

### Example 17 Evaluation of nuclease resistance

For oligonucleotide of the antisense strands 1 and 2 synthesized in Example 15, resistance to exonuclease that decompose from a 3' side was researched. Oligonucleotide (5'-TTTTTTTTTT-3') was used as a control.

A buffer solution (65 µL) containing 750 pmol of the oligonucleotide was kept at 37°C for 5 min, then mixed with a buffer solution containing 5 µg/mL of snake venom phosphodiesterase (Crotalus admanteus venom phosphodiesterase(CAVP): Merck KGaA, Darmstadt, Germany) (35 µL). Degradation of the oligonucleotide was measured over time by HPLC (Thermo UltiMate 3000, analytical column: Waters XBridge^{™} Shield RP18, 3.5 µm, 4.6 mm × 150 mm). A buffer solution (final concentration) was composed of 50 mM Tris-HCl (pH 8.0) and 10 mM MgCl₂, and was fully degassed before measurement. Quantitative conditions of HPLC were as follows:

### [HPLC quantitative conditions]

Mobile phase: Solution A, 0.1M triethylammonium acetate buffer, pH 7.0
Solution B, 0.1M triethylammonium acetate buffer : acetonitrile = 1:1 (v/v), pH 7.0
Gradient: 15-40% (v/v) Solution B (7 min)
Column used: Waters XBridge^{™} Shield RP18 3.5µm (4.6mm×150mm )
Flow rate: 1.0 mL/min
Column temperature: 50°C
Detection: UV (268 nm)

Results were shown in FIG. 1. In FIG. 1, "residual oligonucleotide (%)" represented a remaining rate of undecomposed oligonucleotide (10 mer) at a measurement time point relative to the undecomposed oligonucleotide (10 mer) at time 0.

Results in FIG. 1 indicated that oligonucleotide containing the carbocyclic nucleoside Compound X in the present disclosure at the 3'-terminal had more than 70% unreacted even after 40 min of nuclease treatment, proving that the oligonucleotide was difficult to decompose. Oligonucleotide containing Compound X at a 3^{rd} position from the 3'-terminal had more than 10% unreacted even after 5 min of nuclease treatment. In contrast, oligonucleotide containing all-natural thymidine was almost completely degraded after 5 min of nuclease treatment. Therefore, oligonucleotide containing the carbocyclic nucleoside Compound X in the present disclosure exhibited higher nuclease resistance than that of oligonucleotide containing the all-natural thymidine.

### Industrial Applicability

Conformationally locked carbon-ring nucleoside and nucleotide are provided. The oligonucleotide containing the conformationally locked carbon-ring nucleoside and nucleotide exhibit high binding affinity and nuclease resistance, and are expected to be applied to nucleic acid drugs.

## Claims

1. A compound or salt thereof represented by the following Formula (I): 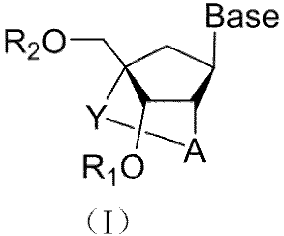
in the formula, R₁ and R₂ independently represent a hydrogen atom, an alkyl group containing 1-6 carbon atoms, a phenyl group, a (C₁₋₃) alkyl-phenyl group, a heterocycle, or a (C₁₋₃) alkyl-heterocycle, a hydroxyl protecting group, a silicon group containing one or more substituents, a phosphate group containing a protecting group for oligonucleotide synthesis, phosphoester, thiophosphate, chiral thiophosphate, phosphate triester, aminoalkyl phosphate triester, and alkyl phosphate ester, respectively;
A is O, S, or NR₃; R₃ is a hydrogen atom, an alkyl group containing 1-6 carbon atoms, a halogenated alkyl group containing 1-6 carbon atoms, -C(O)R₄, -C(O)OR₄, or -C(O)N(R₄)₂; and R₄ is H, or an alkyl group containing 1-6 carbon atoms or an halogenated alkyl group containing 1-6 carbon atoms;
Y is C=O, CHR₅, or CHOR₅; and R₅ is H, or an alkyl group containing 1-6 carbon atoms or a halogenated alkyl group containing 1-6 carbon atoms; and
Base is a base.

2. The compound or salt thereof according to claim 1, wherein Formula (I) is represented by any one of the following Formulae (I-1) - (I-4):

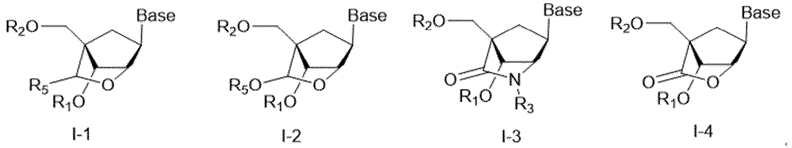

3. The compound or salt thereof according to claim 1, wherein Base is selected any of the following groups:

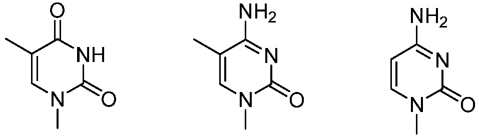

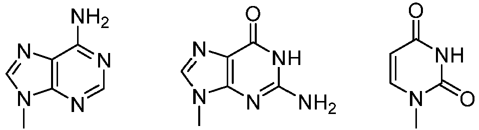

4. The compound or salt thereof according to claim 1, wherein the alkyl group containing 1-6 carbon atoms is a straight-chain alkyl group, a branched alkyl group, a cycloalkyl group or a (C₁₋₄) alkyl group-(C₃₋₇) cycloalkyl group.

5. The compound or salt thereof according to claim 1, wherein the compound represented by Formula (I) has any of the structures shown in the following formula:

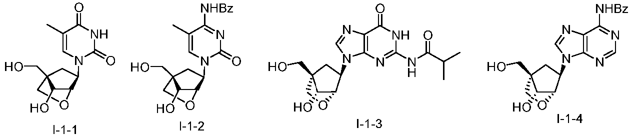

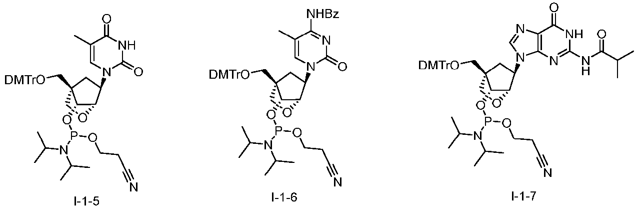

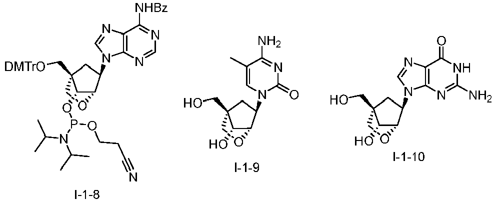

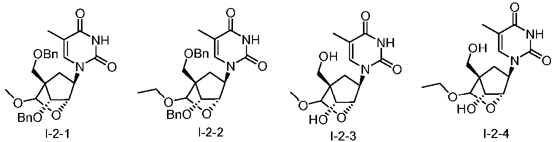

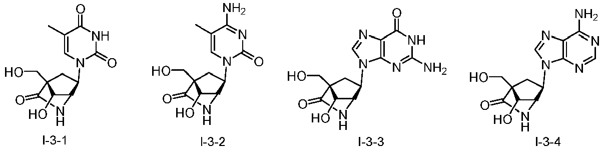

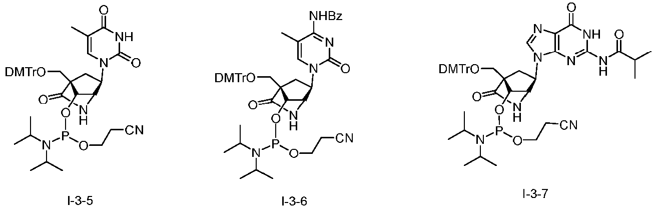

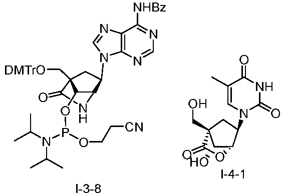

6. An oligonucleotide or pharmaceutically acceptable salt thereof, wherein it contains at least one of the structures shown in Formulae (II)-(IV): 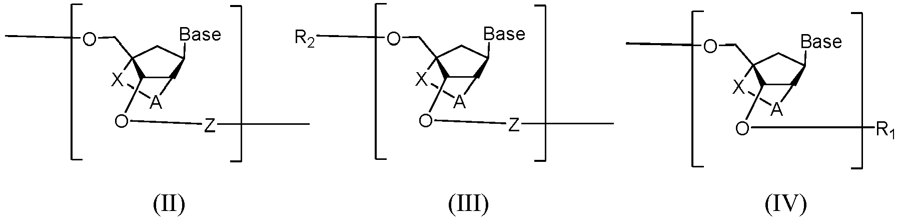 in the formulae, Z is phosphoester, thiophosphate, chiral thiophosphate, phosphate triester, aminoalkyl phosphate triester, and alkyl phosphate ester.

7. A preparation method for the oligonucleotide or pharmaceutically acceptable salt thereof according to claim 6, comprising steps of synthesizing the oligonucleotide using the compound represented by Formula (I) or the pharmaceutically acceptable salt thereof.

8. A preparation method for the oligonucleotide or pharmaceutically acceptable salt thereof according to claims 6-7, comprising steps of synthesizing the oligonucleotide using at least one of the compounds described in claim 5 or the pharmaceutically acceptable salt thereof.

9. A pharmaceutical composition, comprising a therapeutically effective amount of one or more of the compound or salt thereof according to any one of claims 1-5, as well as a pharmaceutically acceptable excipient.

10. Use of the compound or salt thereof according to any one of claims 1-5 in the preparation of a drug for gene therapy, gene vaccination, antisense therapy, interfering RNA, or nucleic acid transfer.

11. A pharmaceutical composition, comprising a therapeutically effective amount of one or more of the oligonucleotide or pharmaceutically acceptable salt thereof according to any one of claims 6-8, as well as a pharmaceutically acceptable excipient.

12. Use of the oligonucleotide or pharmaceutically acceptable salt thereof according to any one of claims 6-8 in the preparation of a drug for gene therapy, gene vaccination, antisense therapy, interfering RNA, or nucleic acid transfer.
